# EUROPEAN PATENT APPLICATION

(11) **EP 4 851 197 A1**
(43) Date of publication of application: **09.12.2026**
(21) Application number: 25748022.8
(22) Date of filing: 27.01.2025
(51) Int. Cl.: C07K 16/46, C07K 16/28, A61K 39/395, A61P 35/00

(54) **CONJUGATED COMPOUND, AND PREPARATION AND USE THEREOF**

(30) Priority: 04.02.2024 CN 202410156442
(71) Applicant: Yichen Therapeutics Limited, Wan Chai, Hong Kong (HK)
(72) Inventor: WANG, Feng, Nantong, Jiangsu 226002 (CN); ZHANG, Yuhan, Nantong, Jiangsu 226002 (CN)
(74) Representative: Studio Torta S.p.A.
(86) International application number: PCT/CN2025/075554
(87) International publication number: WO 2025/162460

(57) **Abstract**

The present invention provides a conjugate compound comprising an antibody moiety and a heterologous moiety, wherein the antibody moiety comprises a first polypeptide chain comprising, from N-terminus to C-terminus, binding modules A1, B1, C1, D1, and E1, and a second polypeptide chain comprising, sequentially from N-terminus to C-terminus, binding modules A2, B2, C2, D2, and E2, and wherein the heterologousmoiety is conjugated to cysteine residues (Cys) in a first hinge region located between modules C1 and D1 and/or in a second hinge region located between modules C2 and D2. By means of non-covalent interactions between A1/A2, B1/B2, C1/C2, D1/D2, and E1/E2, a conjugate compound with stable structure and highly uniform DAR is obtained. The present invention further provides the preparation and use of the conjugate compound.

## Description

### Technical Field

The present invention mainly relates to the field of biopharmaceuticals, and in particular to a conjugate compound, as well as the preparation and use thereof.

### Background Art

Antibody-drug conjugates (ADCs) utilize linkers to connect monoclonal antibodies with small molecule drugs, and use the specificity of the monoclonal antibody to accurately act on a target, thereby reducing the toxic side effects of the drug on normal cells. ADCs combine the specificity and stability of antibody drugs with the pharmacodynamic properties of small-molecule toxins against tumor cells, and are currently one of the major research directions for anti-tumor drugs.

Conventional ADCs are constructed by reacting amino groups of lysine residues in an antibody with succinimide esters on a linker, or by reacting thiol groups obtained after reduction of cysteine residues with maleimide groups on a linker. One antibody molecule contains 80-90 lysine residues, and conjugation may occur at nearly 40 different lysine residues. Opening cysteine disulfide bonds produces multiple cysteine residues and at the same time damages the integrity of the antibody molecule. Therefore, conventional ADCs are highly heterogeneous mixtures, having different drug-to-antibody ratios (DARs) and different conjugation sites, poor stability, and a tendency to aggregate, which affects efficacy and the therapeutic window. In addition, analytical characterization and control of differences between production batches are technically difficult. Site-specific conjugation technology can achieve site-specific and quantitative conjugation of an antibody and a small-molecule toxin. ADCs obtained by this technology have an appropriate DAR, high homogeneity, good stability, good batch-to-batch reproducibility, and better activity and pharmacokinetic properties, and are also more suitable for large-scale production. Site-specific conjugation technology generally requires engineering or modification of an antibody, for example, introducing into the antibody a mutant amino acid containing a thiol group or an unnatural amino acid having an active functional group, so as to achieve site-specific drug conjugation. Selection of the introduction site, selection of the type of introduced amino acid, and the effects of the introduced amino acid on antibody structure and activity impose substantial requirements and challenges. It is particularly important to use the natural sequence of an antibody for site-specific conjugation while maintaining stability and activity of the conjugate on the premise of obtaining a conjugate having a uniform DAR.

### Summary of the Invention

The main objective of the present invention is to provide a conjugate compound having a stable structure and uniform product, so as to solve the problems in the prior art of ADC drugs, such as non-uniform drug-to-antibody ratio (DAR), unstable products, thereby affecting ADC application and druggability.

To achieve the above objective, in a first aspect, the present invention provides a conjugate compound comprising an antibody moiety and a heterologous moiety, wherein the antibody moiety comprises two polypeptide chains:
a) a first polypeptide chain comprising, sequentially from N-terminus to C-terminus, binding modules A1, B1, C1, D1, and E1; and
b) a second polypeptide chain comprising, sequentially from N-terminus to C-terminus, binding modules A2, B2, C2, D2, and E2;
wherein,
the C1 binding module is a heavy chain constant region CH1;
the C2 binding module is a light chain constant region CL, and the cysteine (Cys) at position 214 of the CL is deleted (C214del, EU numbering; all EU numbering hereinafter) or mutated to serine (Ser) (C214S), glycine (Gly) (C214G), or threonine (Thr) (C214T);
the D1 binding module is a first Fc;
the D2 binding module is a second Fc;
the B1 and/or B2 binding modules are each independently selected from a heavy chain variable region (VH), a light chain variable region (VL), a single domain antibody, a VHH domain, a receptor ligand-binding domain, a ligand receptor-binding domain, or a non-immunoglobulin antigen-binding scaffold;
the A1 and/or A2 binding modules are absent or are each independently selected from a heavy chain variable region (VH), a light chain variable region (VL), a single domain antibody, a VHH domain, a receptor ligand-binding domain, a ligand receptor-binding domain, a non-immunoglobulin antigen-binding scaffold, or a single variable domain of a TCR;
the E1 and/or E2 binding modules are absent or are each independently selected from a heavy chain variable region (VH), a light chain variable region (VL), a single domain antibody, a VHH domain, a receptor ligand-binding domain, a ligand receptor-binding domain, or a non-immunoglobulin antigen-binding scaffold;
the C1 binding module and the D1 binding module are connected via a first linker peptide, and the C2 binding module and the D2 binding module are connected via a second linker peptide;
the first linker peptide and the second linker peptide comprise one or more cysteine residues (Cys), and the heterologous moiety is conjugated to the Cys residues.

In a specific embodiment, one of the B1 and B2 binding modules is selected from VH, and the other of the B1 and B2 binding modules is selected from VL, and the VH and VL form a first binding domain.

In a specific embodiment, one of the A1 and A2 binding modules is selected from VH2, and the other of the A1 and A2 binding modules is selected from VL2, and the VH2 and VL2 form a second binding domain.

In a specific embodiment, one of the E1 and E2 binding modules is selected from VH3, and the other of the E1 and E2 binding modules is selected from VL3, and the VH3 and VL3 form a third binding domain.

In a specific embodiment, A1, A1, E1, and E2 are absent, and one of the B1 and B2 binding modules is VH, and the other of the B1 and B2 binding modules is VL, and the VH and VL form a first antigen-binding domain.

In a specific embodiment, E1 and E2 are absent, and one of the B1 and B2 binding modules is VH, and the other of the B1 and B2 binding modules is VL, and one of the A1 and A2 binding modules is VH2, and the other of the A1 and A2 binding modules is VL2, wherein the VH and VL form a first antigen-binding domain, and the VH2 and VL2 form a second antigen-binding domain.

In a specific embodiment, A1 and A2 are absent, and one of the B1 and B2 binding modules is VH, and the other of the B1 and B2 binding modules is VL, and one of the E1 and E2 binding modules is VH3, and the other of the E1 and E2 binding modules is VL3, wherein the VH and VL form a first antigen-binding domain, and the VH3 and VL3 form a third antigen-binding domain.

In a specific embodiment, A1, A2, B1, and B2 are absent, and one of the E1 and E2 binding modules is VH3, and the other of the E1 and E2 binding modules is VL3, and the VH3 and VL3 form a third antigen-binding domain.

In a specific embodiment, the antibody in the conjugate compound is a bispecific antibody targeting EGFR and cMet. In a specific embodiment, the first chain of the bispecific antibody targeting EGFR and cMet comprises, sequentially from N-terminus to C-terminus: a heavy chain variable region of an anti-cMet antibody (VH2), a first linker peptide, a heavy chain variable region (VH1) or light chain variable region (VL1) of an anti-EGFR antibody, CH1, a first hinge region, and a first Fc; and the second chain comprises, from N-terminus to C-terminus: a light chain variable region of an anti-cMet antibody (VL2), a second linker peptide, a light chain variable region (VL1) or heavy chain variable region (VH1) of an anti-EGFR antibody, CL, a second hinge region, and a second Fc.

In a specific embodiment, VH2 comprises HCDR1, HCDR2, and HCDR3 as set forth in SEQ ID NO: 53, SEQ ID NO: 54, and SEQ ID NO: 55, respectively; and VL2 comprises LCDR1, LCDR2, and LCDR3 as set forth in SEQ ID NO: 56, SEQ ID NO: 57, and SEQ ID NO: 58, respectively.

In a specific embodiment, the amino acid sequences of VH2 and VL2 are as set forth in SEQ ID NO: 38 and SEQ ID NO: 40, respectively.

In a specific embodiment, the HCDR1, HCDR2, and HCDR3 of VH1 are selected from the group consisting of SEQ ID NO: 59-61, SEQ ID NO: 65-67, or SEQ ID NO: 71-73, respectively; and the LCDR1, LCDR2, and LCDR3 of VL1 are selected from the group consisting of SEQ ID NO: 62-64, SEQ ID NO: 68-70, or SEQ ID NO: 74-76, respectively.

In a specific embodiment, the amino acid sequences of VH1 and VL1 are as set forth in SEQ ID NO: 42 and SEQ ID NO: 44, SEQ ID NO: 46 and SEQ ID NO: 48, or SEQ ID NO: 50 and SEQ ID NO: 52, respectively.

In a specific embodiment, the two chains of the bispecific antibody targeting EGFR and cMet have amino acid sequences as set forth in: SEQ ID NO: 1 and SEQ ID NO: 2; SEQ ID NO: 3 and SEQ ID NO: 4; SEQ ID NO: 5 and SEQ ID NO: 6; SEQ ID NO: 7 and SEQ ID NO: 8; SEQ ID NO: 9 and SEQ ID NO: 10; SEQ ID NO: 11 and SEQ ID NO: 12; SEQ ID NO: 77 and SEQ ID NO: 78; SEQ ID NO: 79 and SEQ ID NO: 80; SEQ ID NO: 81 and SEQ ID NO: 82; SEQ ID NO: 83 and SEQ ID NO: 84; SEQ ID NO: 85 and SEQ ID NO: 86; SEQ ID NO: 87 and SEQ ID NO: 88; SEQ ID NO: 89 and SEQ ID NO: 90; SEQ ID NO: 91 and SEQ ID NO: 92; SEQ ID NO: 93 and SEQ ID NO: 94; SEQ ID NO: 95 and SEQ ID NO: 96; SEQ ID NO: 117 and SEQ ID NO: 118; SEQ ID NO: 119 and SEQ ID NO: 120; SEQ ID NO: 121 and SEQ ID NO: 121; SEQ ID NO: 123 and SEQ ID NO: 124; SEQ ID NO: 125 and SEQ ID NO: 126; SEQ ID NO: 127 and SEQ ID NO: 128; SEQ ID NO: 129 and SEQ ID NO: 130; SEQ ID NO: 131 and SEQ ID NO: 132; SEQ ID NO: 133 and SEQ ID NO: 134; or SEQ ID NO: 135 and SEQ ID NO: 136.

In a specific embodiment, the first linker peptide and the second linker peptide are polypeptides having a length of 5 to 20 amino acids.

In a specific embodiment, the first linker peptide and the second linker peptide may be the same or different. In a specific embodiment, the first linker peptide and the second linker peptide are flexible peptides; further, the flexible peptide comprises glycine (Gly) and/or serine (Ser); further, the flexible peptide comprises glycine (Gly), serine (Ser), threonine (Thr), alanine (Ala), glutamic acid (Glu), and/or phenylalanine (Phe). In a specific embodiment, the first linker peptide and the second linker peptide are rigid peptides; further, the rigid peptide consists of an α-helix; further, the rigid peptide comprises glutamic acid (Glu), alanine (Ala), and/or lysine (Lys).

In a specific embodiment, the first linker peptide and the second linker peptide comprise the amino acid sequence as set forth in SEQ ID NO: 137: EPKSCDKTHTCPPCP; further, one or two Cys residues at positions 220, 226, and/or 229 in SEQ ID NO: 137 of the first linker peptide and the second linker peptide are deleted and/or mutated; further, the one or two Cys residues at positions 220, 226, and/or 229 in SEQ ID NO: 137 of the first linker peptide and the second linker peptide are mutated to Ser, Thr, or Gly; further, the first linker peptide and the second linker peptide comprise the amino acid sequence as set forth in SEQ ID NO: 138, 139, 140, 141, 142, 143, 144, 145, 146, 147, 148, 149, 166, 167, 168, 169, 170, or 171.

In a specific embodiment, the first linker peptide and the second linker peptide are the hinge of IgG2; further, the first linker peptide and the second linker peptide comprise the amino acid sequence as set forth in SEQ ID NO: 156: ERKCCVECPPCP (E216-P230); further, one or two Cys residues at positions 219, 220, 226, and/or 229 in SEQ ID NO: 156 of the first linker peptide and the second linker peptide are deleted and/or mutated; further, the Cys is mutated to Ser, Thr, or Gly; further, the first linker peptide and the second linker peptide comprise the amino acid sequence as set forth in SEQ ID NO: 157, 158, 159, 160, 161, 162, 163, or 164.

In a specific embodiment, the first linker peptide and the second linker peptide are the hinge of IgG4; further, the first linker peptide and the second linker peptide comprise the amino acid sequence as set forth in SEQ ID NO: 150: ESKYGPPCPPCP (E216-P230); further, one of the Cys residues at positions 226 and 229 in SEQ ID NO: 150 of the first linker peptide and the second linker peptide is deleted or mutated; further, the Cys is mutated to Ser, Gly, or Thr; further, the first linker peptide and the second linker peptide comprise the amino acid sequence as set forth in SEQ ID NO: 151, 152, 154, or 155.

In a specific embodiment, the first linker peptide and the second linker peptide are the hinge of IgG3; further, the first linker peptide and the second linker peptide comprise the amino acid sequence as set forth in SEQ ID NO: 165: ELKTPLGDTTHTCPRCP(EPKSCDTPPPCPRCP)₃.

In a specific embodiment, the first Fc and the second Fc comprise modified CH3 domains, wherein the modified CH3 domains comprise amino acid substitutions that promote heterodimerization of the first Fc and the second Fc. In a specific embodiment, one of the first Fc and the second Fc comprises the amino acid substitution T366W, and the other of the first Fc and the second Fc comprises the amino acid substitutions T366S, L368A, and Y407V. In a specific embodiment, the one of the first Fc and the second Fc comprising the amino acid substitution T366W further comprises the amino acid substitution S354C, and the other of the first Fc and the second Fc comprising the amino acid substitutions T366S, L368A, and Y407V further comprises the amino acid substitution Y349C. In a specific embodiment, one of the first Fc and the second Fc comprises the amino acid substitutions E356K and R409K, and the other of the first Fc and the second Fc comprises the amino acid substitutions R409K and K439E.

In a specific embodiment, the heterologous moiety of the conjugate compound is a substance suitable for tumor targeting, disease diagnosis, cure, alleviation, treatment, or prevention. Preferably, the heterologous moiety is a drug, toxin, CDK inhibitor, HDAC inhibitor, TLR agonist, PROTAC protein degrader, radionuclide, immunomodulator, glutamate urea (DUPA) or a analog thereof, cytokine, lymphokine, chemokine, growth factor, tumor necrosis factor, hormone, hormone antagonist, enzyme, oligonucleotide, DNA, RNA, siRNA, RNAi, microRNA, peptide nucleic acid, phototherapeutic agent, anti-angiogenic agent, pro-apoptotic agent, non-natural amino acid, peptide, lipid, carbohydrate, scaffold molecule, fluorescent label, visualization peptide, biotin, serum half-life modifier, capture tag, chelator, or a combination thereof.

In a specific embodiment, the heterologous moiety of the conjugate compound is selected from an ethyleneimine derivative, triazene, folic acid analog, anthracycline, taxane, COX-2 inhibitor, pyrimidine analog, purine analog, antibiotic, enzyme inhibitor, epipodophyllotoxin, platinum coordination compound, vinca alkaloid, substituted urea, adrenocortical suppressant, hormone antagonist, endostatin, camptothecin, camptothecin derivative, SN-38, doxorubicin, doxorubicin analog, antimetabolite, alkylating agent, antimitotic agent, anti-angiogenic agent, mTOR inhibitor, heat shock protein inhibitor, proteasome inhibitor, HDAC inhibitor, pro-apoptotic agent, methotrexate, CPT-11, or a combination thereof.

In a specific embodiment, the drug in the conjugate compound is selected from a nitrogen mustard, alkyl sulfonate, nitrosourea, gemcitabine, methylhydrazine derivative, paclitaxel, tyrosine kinase inhibitor, or a combination thereof. In a specific embodiment, the drug is selected from auristatin, monomethyl auristatin E (MMAE), monomethyl auristatin F (MMAF), Dinaciclib, Mocetinostat, vorinostat, olaparib, Ceralasertib, M3541, CC-885, Dx-8951, tubulysin, exatecan derivative (Dxd), pyrrolobenzodiazepine (PBD), maytansinoid, DM1, DM4, calicheamicin, duocarmycin (CAS NO. 130288), duostatin, duostatin-3, duostatin-5, rachelmycin (CC-1065), SN-38, SG3199, or doxorubicin.

In a specific embodiment, the heterologous moiety in the conjugate compound is selected from glutamate urea (DUPA).

In a specific embodiment, the heterologous moiety in the conjugate compound is conjugated to the Cys residues of the first linker peptide and/or the second linker peptide via a linker.

In a specific embodiment, the linker in the conjugate compound is selected from a peptide linker, a non-peptide linker, a cleavable linker, or a non-cleavable linker.

In a specific embodiment, the linker in the conjugate compound is selected from mc (maleimidocaproyl), val-cit (valine-citrulline), mc-val-cit (maleimidocaproyl-valine-citrulline), mc-val-cit-PABC (maleimidocaproyl-valine-citrulline-p-aminobenzyl carbamate), mcGGFG, mc-(PEG)₈-VA-PABC, MCC (N-maleimidomethyl cyclohexane-1-carboxylate), Mal-PEG2C₂ (maleimido-[CH2CH2O]2CH2CH2C(=O)), Mal-PEG3C₂ (maleimido-[CH2CH2O]3CH2CH2C(=O)), and Mal-PEG6C₂ (maleimido-[CH2CH2O]6CH2CH2C(=O)).

To address the issue of batch-to-batch quality heterogeneity caused by thioether bond formation in antibody fusion proteins during production and storage, as well as the efficacy and safety risks (e.g., greater immunogenic potential) associated with thioether bond formation during in vivo therapy, the present invention provides a fusion antibody. By modifying cysteine residues (Cys) at specific sites on the hinges of the two polypeptide chains constituting the fusion antibody, no inter-chain thioether product is observed after expression.

In another aspect, the present invention provides a method for preparing the conjugate compound described above, comprising placing the antibody moiety under reducing conditions so that thiol groups of one or more Cys residues in the first and second linking peptides are reduced, and then reacting the reduced thiol groups with an active agent; preferably, the thiol groups are reacted with the heterologous moiety via a Michael reaction using a linker comprising a maleimide group.

In another aspect, the present invention provides a composition comprising the conjugate compound described above, and a pharmaceutically acceptable carrier, diluent, or excipient.

In another aspect, the present invention provides use of the composition described above in the manufacture of a medicament for the prevention or treatment of a disease. Further, the disease to be prevented or treated includes, but is not limited to, cancer (e.g., epithelial cell carcinoma, breast cancer, ovarian cancer, lung cancer, small cell lung cancer, prostate cancer, colon cancer, rectal cancer, bladder cancer, kidney cancer, liver cancer, thyroid cancer, endometrial cancer, pharyngeal cancer, nasal cancer, pancreatic cancer, skin cancer, tongue cancer, esophageal cancer, vaginal cancer, cervical cancer, splenic cancer, testicular cancer, gastric cancer, thymic cancer, hepatocellular carcinoma, or sporadic or hereditary papillary renal cell carcinoma, muscle cancer, bone cancer, mesothelioma, vascular cancer, fibrotic cancer, leukemia, or lymphoma), autoimmune disease, inflammatory disease, or infectious disease

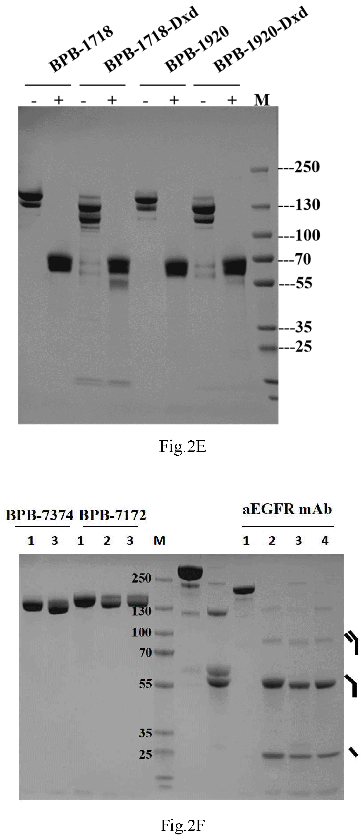

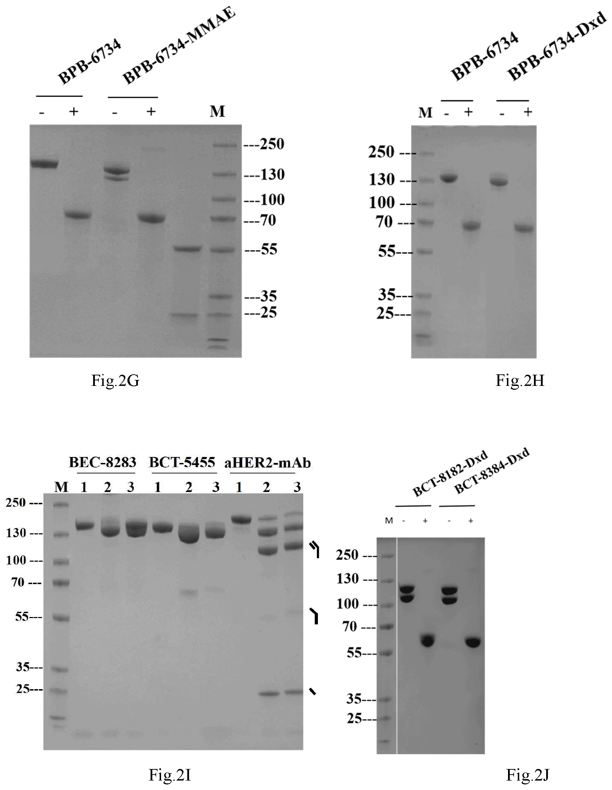

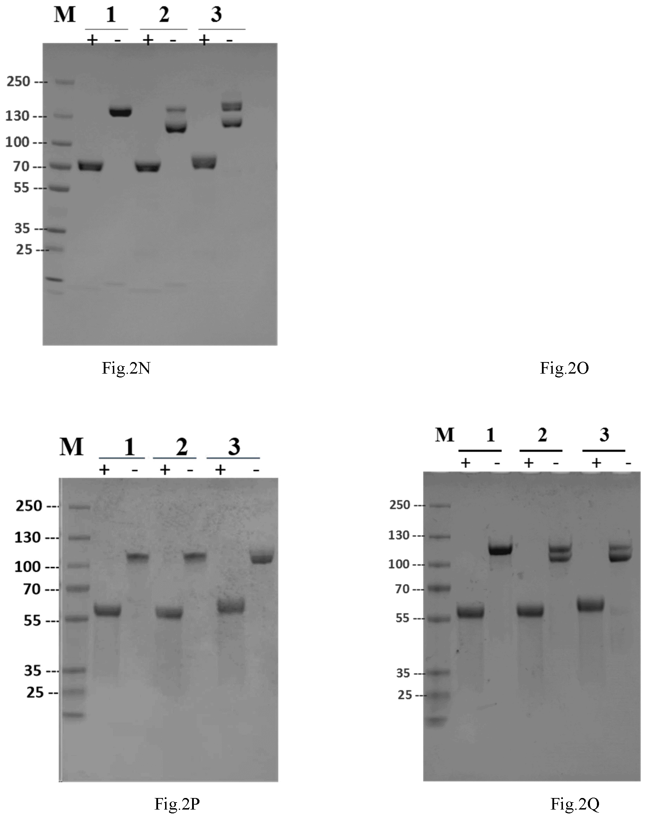

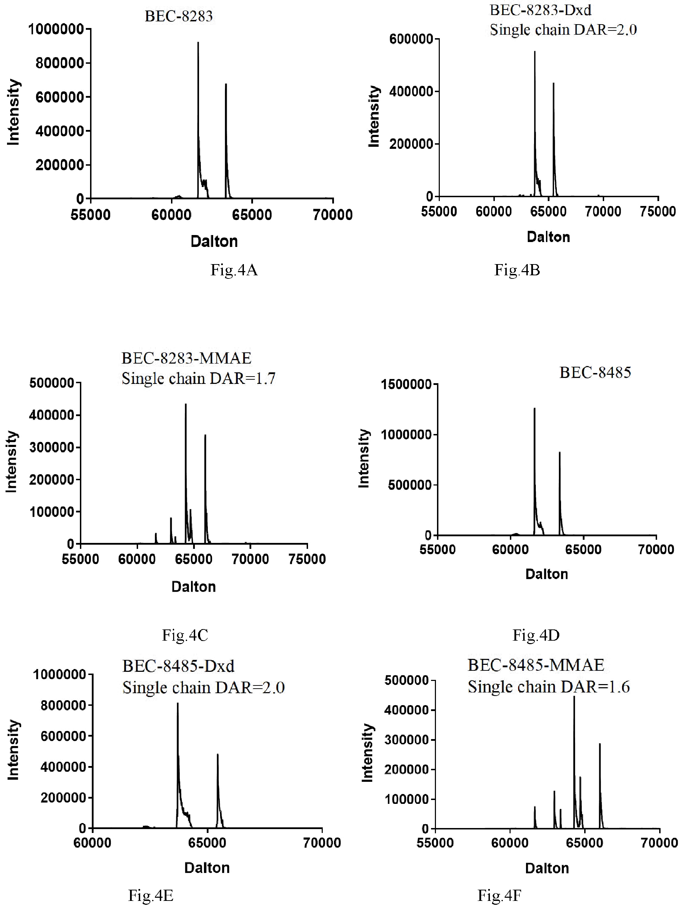

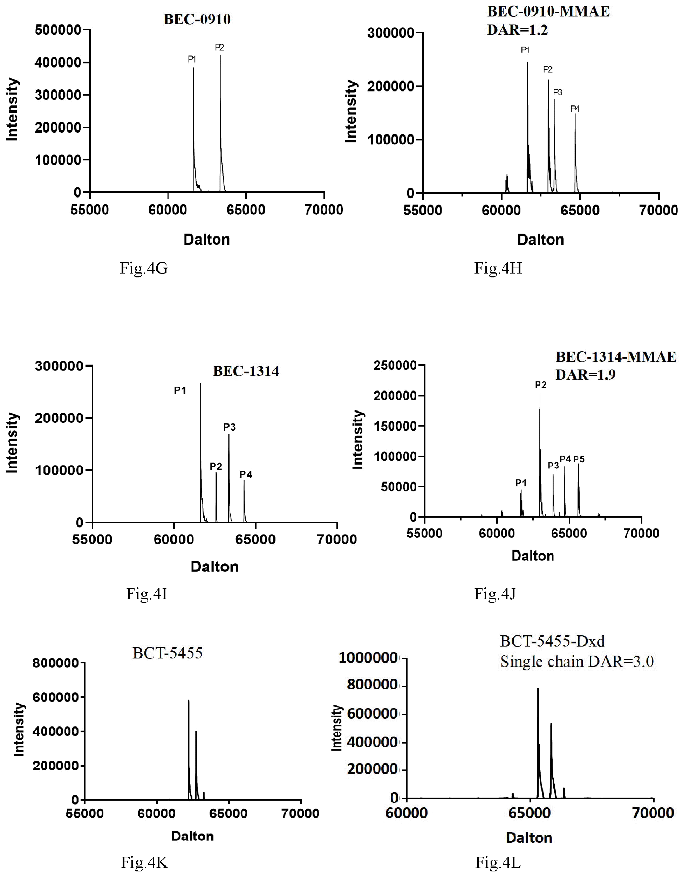

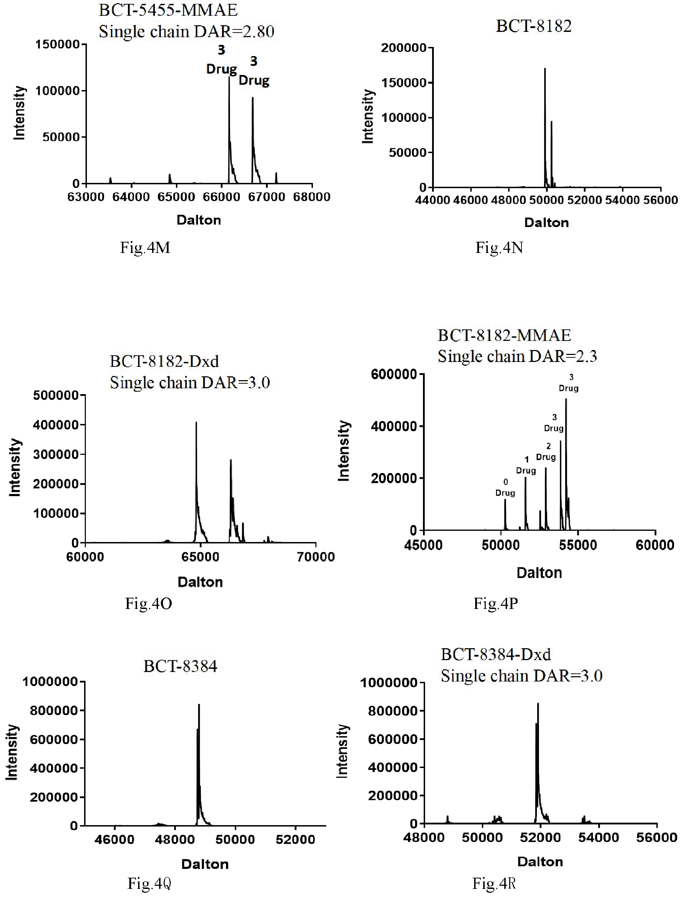

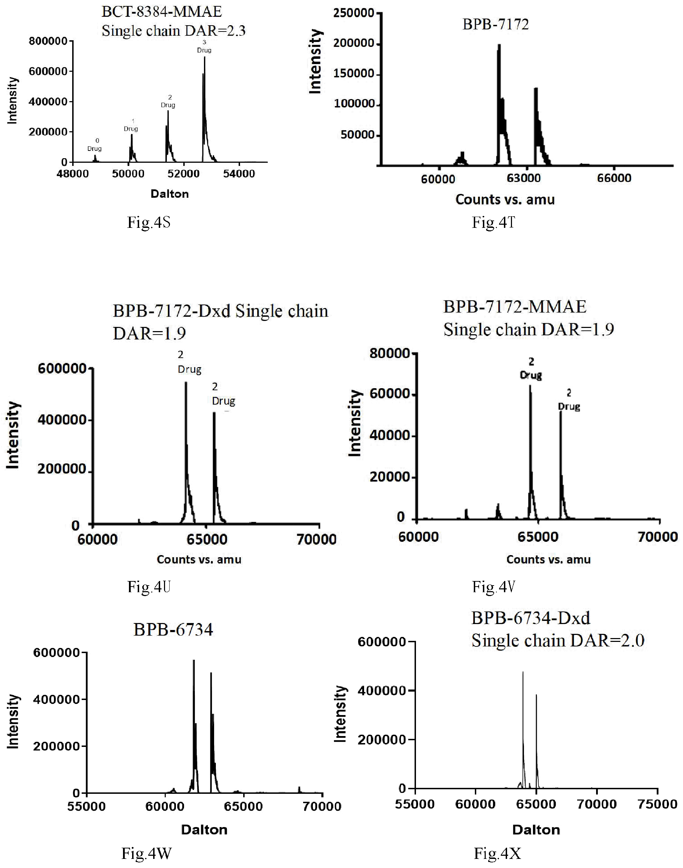

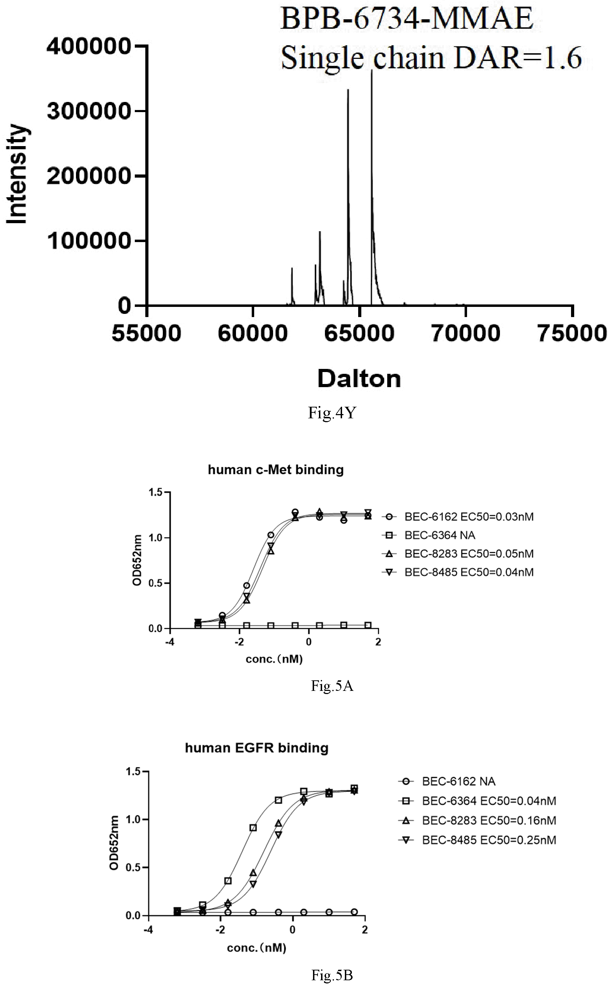

The present invention has discovered that, after disulfide bonds of Cys residues in the first and second linker peptides of the antibody moiety are opened using the reducing agent TCEP (tris(2-carboxyethyl)phosphine hydrochloride), the heterologous moiety can be conjugated to the reduced thiol groups. By means of the non-covalent VH/VL interaction formed by the B1 and B2 binding modules, the non-covalent interaction between the C1 binding module (CH1) and the C2 binding module (CL), and heterologous pairing between the D1 binding module (first Fc) and the D2 binding module (second Fc), the resulting conjugated compound has a stable structure, as shown by bands between 100 and 130 kDa in the non-reducing SDS-PAGE of Figures 2P and 2Q. Further non-covalent VH2/VL2 interaction formed by the additional A1 and A2 binding modules further enhances stability, as shown by bands near 130 kDa in Figures 2F, 2I, 2N and 2O. By contrast, conjugated compounds obtained using other marketed ADC conjugation technologies appear as multiple bands under non-reducing SDS-PAGE, as shown in lanes 2-4 for the anti-EGFR mAb in Figure 2F and lanes 2-3 for the anti-HER2 mAb in Figure 2I. In addition, because the Cys residues involved in conjugation are located in the linker peptides and have similar reactivity, the resulting conjugated compounds have DAR values that are more uniform than those of other conjugated compounds, as shown in Figure 4, which is advantageous for ensuring consistency of clinical efficacy and safety.

### Brief Description of the Drawings

Figure 1 is a schematic diagram showing the structure of the antibody moiety of the conjugate compound.
Figures 2A-2Q are SDS-PAGE gel images of the antibodies and their conjugates, wherein "-" and "+" indicate the presence or absence of reducing agent, respectively. In Figures 2F and 2I, samples were treated under non-reducing conditions. "1" represents a antibody treated with Tcep (tris(2-carboxyethyl)phosphine hydrochloride), "2" represents a Dxd-conjugated antibody treated with Tcep; lane "3" represents a MMAE-conjugated antibody treated with Tcep; and lane "4" represents a Dx8951-conjugated antibody treated with Tcep.
Figure 2K is an SDS-PAGE gel image obtained in the presence of the reducing agent DTT, wherein lanes 1-10 are MTE-6263, MTE-6465, MTE-6667, MTE-6869, MTE-7071, MTE-7273, MTE-7475, MTE-7677, MTE-7879, and MTE-8081, respectively.
Figure 2L is an SDS-PAGE gel image obtained in the presence of the reducing agent DTT, wherein lanes 1-11 are BEC-2728, BEC-8283, BEC-2930, BEC-3132, BEC-3334, BEC-3536, BEC-3738, BEC-3940, BEC-4142, BEC-4344, and BEC-4546, respectively.
Figure 2M is an SDS-PAGE gel image obtained in the presence of the reducing agent DTT, wherein lanes 1-10 are BEC-9293, BEC-9495, BEC-9697, BEC-9899, BEC-0001, BEC-0203, BEC-0405, BEC-0607, BEC-0809, and BEC-1011, respectively.
Figure 2N shows BEC-9293 and its conjugates, wherein "1" is BEC-9293, "2" is BEC-9293 after Tcep treatment, and "3" is the DUPA-conjugated BEC-9293 after Tcep treatmentp.
Figure 2O shows BEC-9495 and its conjugates, wherein "1" is BEC-9495, "2" is BEC-9495 after Tcep treatment, and "3" is the DUPA-conjugated BEC-9495 after Tcep treatment.
Figure 2P shows MTE-6465 and its conjugates, wherein "1" is MTE-6465, "2" is MTE-6465 after Tcep treatment, and "3" is the DUPA-conjugated MTE-6465 after Tcep treatment.
Figure 2Q shows MTE-9091 and its conjugates, wherein "1" is MTE-9091, "2" is MTE-9091 after Tcep treatment, and "3" is the DUPA-conjugated MTE-9091 after Tcep treatment.
Figure 2R shows MTE-8687 and its conjugates, wherein "1" is MTE-8687, "2" is MTE-8687 after Tcep treatment, and "3" is the DUPA-conjugated MTE-8687 after Tcep treatment.
Figures 3A and 3B show size exclusion chromatography of the bispecific antibodies and their conjugates.
Figures 4A-4Y show mass spectrometry analysis of MMAE- or Dxd-based conjugates.
Figures 5A-5T show the results of ELISA-based binding activity of the bispecific antibodies and their conjugates to antigens.
Figures 6A-6D show flow cytometric analysis of the binding of the bispecific antibodies to cell surface antigens.
Figures 7A-7B show internalization mediated by bispecific antibodies in different cell lines.
Figures 8A-8K show the specific cytotoxicity of MMAE-based bispecific antibody-drug conjugates against cells.
Figure 9 shows the specific cytotoxicity of Dxd-based bispecific antibody-drug conjugates against cells.
Figures 10A and 10B show, respectively, the tumor-inhibitory effect of BEC-8283 and an MMAE conjugate thereof in NCI-H1975 tumor-bearing mice and the effect thereof on body weight.
Figures 11A and 11B show the tumor inhibitory effect of BEC-8283 and its Dxd conjugate on HCC827 tumor-bearing mice, and the effect on body weight, respectively.

### Detailed Description

The present invention is described in detail herein by reference to the following definitions and examples. The contents of all patents and publications mentioned herein, including all sequences disclosed in such patents and publications, are expressly incorporated by reference.

As used herein, "conjugation" and "conjugate" have the same meaning and are used interchangeably herein. A "conjugate compound" comprises an antibody moiety and a heterologous moiety, wherein the antibody moiety comprises two polypeptide chains: a first polypeptide chain comprising, sequentially from N-terminus to C-terminus, binding modules A1, B1, C1, D1, and E1; and a second polypeptide chain comprising, from N-terminus to C-terminus, binding modules A2, B2, C2, D2, and E2, wherein:
the C1 binding module is a heavy chain constant region CH1;
the C2 binding module is a light chain constant region CL;
the D1 binding module is a first Fc;
the D2 binding module is a second Fc;
the B1 and/or B2 binding modules may be absent or each independently selected from, but not limited to, a heavy chain variable region (VH), a light chain variable region (VL), a single domain antibody, a VHH domain, a receptor ligand-binding domain, a ligand receptor-binding domain, a non-immunoglobulin antigen-binding scaffold, or a single variable domain of a TCR;
the A1 and/or A2 binding modules may be absent oreach independently selected from, but not limited to, a heavy chain variable region (VH), a light chain variable region (VL), a single domain antibody, a VHH domain, a receptor ligand-binding domain, a ligand receptor-binding domain, a non-immunoglobulin antigen-binding scaffold, or a single variable domain of a TCR;
the E1 and/or E2 binding modules may be absent or each independently selected from, but not limited to, a heavy chain variable region (VH), a light chain variable region (VL), a single domain antibody, a VHH domain, a receptor ligand-binding domain, a ligand receptor-binding domain, a non-immunoglobulin antigen-binding scaffold, or a single variable domain of a TCR;
the C1 binding module and the D1 binding module are connected via a first linker peptide, and the C2 binding module and the D2 binding module are connected via a second linker peptide;
the first linker peptide and the second linker peptide comprise one or more cysteine residues (Cys), and the heterologous moiety is conjugated to the Cys residues.

As used herein, the "linker peptide" of the present invention is primarily used to provide Cys residues for conjugation with the heterologous moiety. As needed, the linker peptide may contain one, two, three, or even more Cys residues in order to obtain conjugates with a specific DAR. Thus, the "linker peptide" of the present invention may be a flexible peptide, a rigid peptide, or a combination of flexible and rigid peptides. The "first linker peptide" and "second linker peptide" merely indicate the polypeptide chain on which they are located, and are not intended to limit their sequences. The "first linker peptide" and "second linker peptide" may be the same or different, as long as the Cys residues thereon are capable of forming correct and stable interchain pairing. For example, the "linker peptide" may be a flexible peptide, a rigid peptide, or consist of a flexible peptide and a rigid peptide. Preferably, the linker peptide of the present invention may be selected from the hinge regions of IgG1, IgG2, IgG3, IgG4, or a combination thereof. When selected from the hinge region of IgG1, the linker peptide comprises the sequence as set forth in any one of SEQ ID NO: 137-149, SEQ ID NO: 166-171, or a combination thereof. When selected from the hinge region of IgG2, the linker peptide comprises the sequence as set forth in any one of SEQ ID NO: 156-164, or a combination thereof. When selected from the hinge region of IgG4, the linker peptide comprises the sequence as set forth in any one of SEQ ID NO: 150-155, or a combination thereof. When selected from the hinge region of IgG3, the linker peptide comprises the sequence as set forth in SEQ ID NO: 165.

Due to the homodimeric nature of molecules comprising an Fc region, the first Fc and the second Fc of the present invention comprise modified CH3 domains, wherein the modified CH3 domains comprise amino acid mutations that promote heterodimerization of the first Fc and the second Fc (e.g., chimeric mutations, complementary mutations, lock-and-dock mutations, knobs-into-holes mutations, charge mutations, strand-exchange engineered domain (SEED) mutations, etc.). Accordingly, the first Fc and the second Fc may be selected from the IgG, IgA, IgE, or IgM isotypes. Further, the first Fc and the second Fc may be independently selected from IgG1, IgG2, IgG3, or IgG4.

In a specific embodiment, one of the first Fc and the second Fc comprises the amino acid substitution T366W, and the other of the first Fc and the second Fc comprises the amino acid substitutions T366S, L368A, and Y407V.

In a specific embodiment, one of the first Fc and the second Fc comprises the amino acid substitutions T366W and S354C, and the other of the first Fc and the second Fc comprises the amino acid substitutions Y349C, T366S, L368A, and Y407V.

In a specific embodiment, one of the first Fc and the second Fc comprises the amino acid substitutions E356K and R409K, and the other of the first Fc and the second Fc comprises the amino acid substitutions R409K and K439E.

In a specific embodiment, one of the first Fc and the second Fc comprises the amino acid substitution K409R, and the other of the first Fc and the second Fc comprises the amino acid substitution L368E.

In a specific embodiment, one of the first Fc and the second Fc comprises the amino acid substitution F405L, and the other of the first Fc and the second Fc comprises the amino acid substitution K409R.

In a specific embodiment, one of the first Fc and the second Fc comprises the amino acid substitutions S364H and F405A, and the other of the first Fc and the second Fc comprises the amino acid substitutions Y349T and T394F.

In a specific embodiment, one of the first Fc and the second Fc comprises the amino acid substitutions T350V, L351Y, F405A, and Y407V, and the other of the first Fc and the second Fc comprises the amino acid substitutions T350V, T366L, K392L, and T394W.

In a specific embodiment, one of the first Fc and the second Fc comprises the amino acid substitutions K409D and K392D, and the other of the first Fc and the second Fc comprises the amino acid substitutions D399K and E356K.

In a specific embodiment, one of the first Fc and the second Fc comprises the amino acid substitutions K360E and K409W, and the other of the first Fc and the second Fc comprises the amino acid substitutions Q347R, D399V, and F405T.

In a specific embodiment, one of the first Fc and the second Fc comprises the amino acid substitutions F405L, D356E, and L358M, and the other of the first Fc and the second Fc comprises the amino acid substitutions K409R, D356E, and L358M.

In a specific embodiment, one of the first Fc and the second Fc comprises the amino acid substitutions T366S, L368A, Y407V, D356E, and L358M, and the other of the first Fc and the second Fc comprises the amino acid substitutions T366W, D356E, and L358M.

As used herein, the "heterologous moiety" is a substance suitable for tumor targeting, disease diagnosis, cure, alleviation, treatment, or prevention. Preferably, the heterologous moiety is selected from the group consisting of a drug, a toxin, a CDK inhibitor, an HDAC inhibitor, a TLR agonist, a PROTAC protein degrader, a radionuclide, an immunomodulator, glutamate urea (DUPA) and analogs thereof, a cytokine, a lymphokine, a chemokine, a growth factor, a tumor necrosis factor, a hormone, a hormone antagonist, an enzyme, an oligonucleotide, DNA, RNA, siRNA, RNAi, microRNA, a peptide nucleic acid, a phototherapeutic agent, an anti-angiogenic agent, a pro-apoptotic agent, a non-natural amino acid, a peptide, a lipid, a carbohydrate, a scaffold molecule, a fluorescent label, a visualization peptide, biotin, a serum half-life modifier, a capture tag, a chelator, and a solid support.

Preferably, the drug is selected from an ethyleneimine derivative, a triazene, a folic acid analog, an anthracycline, a taxane, a COX-2 inhibitor, a pyrimidine analog, a purine analog, an antibiotic, an enzyme inhibitor, an epipodophyllotoxin, a platinum coordination compound, a vinca alkaloid, a substituted urea, an adrenocortical suppressant, a hormone antagonist, endostatin, camptothecin, a camptothecin derivative, SN-38, doxorubicin, a doxorubicin analog, an antimetabolite, an alkylating agent, an antimitotic agent, an anti-angiogenic agent, an mTOR inhibitor, a heat shock protein inhibitor, a proteasome inhibitor, an HDAC inhibitor, a pro-apoptotic agent, methotrexate, CPT-11, or a combination thereof.

Preferably, the drug is selected from a nitrogen mustard, an alkyl sulfonate, a nitrosourea, gemcitabine, a methylhydrazine derivative, paclitaxel, a tyrosine kinase inhibitor, or a combination thereof.

More preferably, the drug is selected from auristatin, monomethyl auristatin E (MMAE), monomethyl auristatin F (MMAF), Dinaciclib, Mocetinostat, vorinostat, olaparib, Ceralasertib, M3541, CC-885, Dx-8951, tubulysin, exatecan derivative (Dxd), pyrrolobenzodiazepine (PBD), a maytansinoid, DM1, DM4, calicheamicin, duocarmycin (CAS NO. 130288), duostatin, duostatin-3, duostatin-5, rachelmycin (CC-1065), SN-38, SG3199, or doxorubicin.

The "linker" of the present invention may be selected from a cleavable linker, a non-cleavable linker, a peptide linker, or a non-peptide linker. Non-limiting examples of the "linker" of the present invention include mc (maleimidocaproyl), val-cit (valine-citrulline), mc-val-cit (maleimidocaproyl-valine-citrulline), mc-val-cit-PABC (maleimidocaproyl-valine-citrulline-p-aminobenzyl carbamate), mcGGFG, mc-(PEG)₈-VA-PABC, MCC (N-maleimidomethyl cyclohexane-1-carboxylate), Mal-PEG2C₂ (maleimido-[CH₂CH₂O]₂CH₂CH₂C(=O)), Mal-PEG3C₂ (maleimido-[CH₂CH₂O]₃CH₂CH₂C(=O)), or Mal-PEG6C₂(maleimido-[CH₂CH₂O]₆CH₂CH₂C(=O)).

The reducing conditions used in the preparation of the conjugate compound of the present invention are provided by a reducing agent. The interchain disulfide bond reducing agent used in the preparation may be any reagent suitable for reducing interchain cysteine disulfide bonds. Suitable interchain disulfide bond reducing agents are well known to those skilled in the art, see, e.g., R.E. Hansen et al., Analytical Biochemistry, 2009, 394, 147-158. Generally, they are water-soluble and have a negative redox potential at pH 7. The reducing agent may be a thiol or a phosphine. Suitable thiols include 1,4-(dithiobutyl)-2-amine (DTBA), glutathione, cysteine, 2-mercaptoethanol, 2-mercaptoethylamine, dithioerythritol (DTE), or dithiothreitol (DTT). Suitable phosphines include tris(3-sulfophenyl)phosphine, tris(2-hydroxyethyl)phosphine (TCEP), tris(3-hydroxypropyl)phosphine (THPP), or tris(hydroxymethyl)phosphine. Preferred reducing agents are tris(3-sulfophenyl)phosphine, TCEP, and DTT. The most preferred interchain disulfide bond reducing agent is TCEP.

### Examples

The examples are provided for illustration only and are not intended to limit the present invention in any manner.

### Example 1. Vector construction

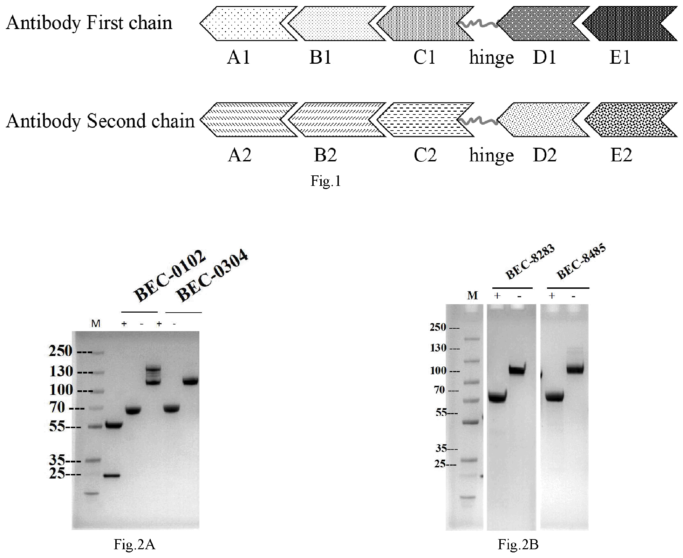

Gene fragments encoding the VL and VH regions of the antibody and gene fragments encoding the antibody constant regions were synthesized separately. The above gene fragments were amplified by PCR. The amplified VH gene fragment was ligated with the antibody constant region gene fragment by overlap PCR to generate the first chain. The amplified VL gene fragment was ligated with the antibody constant region gene fragment to generate the second chain, as shown in Figure 1. The resulting fragments were further ligated by homologous recombination into the pFuse vector (InvivoGen, CA) for eukaryotic expression. The ligated product was transformed into competent E. coli DH5α cells, followed by antibiotic selection on LB plates. Positive clones were selected, and plasmid DNA was extracted using an endotoxin-free plasmid extraction kit. The sequences of the extracted plasmids were verified by sequencing. The nucleotide and amino acid sequences of each construct are as follows:

**Table 1. Amino Acid Sequence List**

| | | | |
|---|---|---|---|
| BEC-8283 (acMet-EGFR) | First chain | SEQ ID NO.1 | hinge2(IgG1 C220del) |
| | Second chain | SEQ ID NO.2 | |
| BEC-8485 (acMet-EGFR) | Second chain | SEQ ID NO.3 | hinge2(IgG1 C220del) |
| | First chain | SEQ ID NO.4 | |
| BEC-0910 (acMet-EGFR) | First chain | SEQ ID NO.5 | hinge3(IgG1 C220del+C226S) |
| | Second chain | SEQ ID NO.6 | |
| BEC-1112 (acMet-EGFR) | First chain | SEQ ID NO.7 | hinge3(IgG1 C220del+C226S) |
| | Second chain | SEQ ID NO.8 | |
| BEC-1314 (acMet-EGFR) | First chain | SEQ ID NO.9 | hinge4: EPKSDKTHTCPPS |
| | Second chain | SEQ ID NO.10 | |
| BEC-1516 (acMet-EGFR) | Second chain | SEQ ID NO.11 | hinge4: EPKSDKTHTCPPS |
| | First chain | SEQ ID NO.12 | |
| BEC-6162 (acMet control) | First chain | SEQ ID NO.27 | hinge2:EPKSDKTHTCPPC |
| | Second chain | SEQ ID NO.28 | |
| BEC-6364 (aEGFR control) | First chain | SEQ ID NO.29 | hinge2(IgG1 C220del) |
| | Second chain | SEQ ID NO.30 | |
| BPB-7172 (aPSMA-aB7H3) | First chain | SEQ ID NO.13 | hinge2(IgG1 C220del) |
| | Second chain | SEQ ID NO.14 | |
| BPB-7374 (aPSMA-aB7H3) | Second chain | SEQ ID NO.15 | hinge2(IgG1 C220del) |
| | First chain | SEQ ID NO.16 | |
| BPB-6734 (aPSMA-aB7H3) | First chain | SEQ ID NO.17 | hinge2(IgG1 C220del) |
| | Second chain | SEQ ID NO.18 | |
| BPB-7576 (aPSMA-aB7H3) | Second chain | SEQ ID NO.19 | hinge2(IgG1 C220del) |
| | First chain | SEQ ID NO.20 | |
| BPB-1718 (aPSMA-aB7H3) | First chain | SEQ ID NO.21 | hinge3(IgG1 C220del+C226S) |
| | Second chain | SEQ ID NO.22 | |
| BPB-1920 (aPSMA-aB7H3) | First chain | SEQ ID NO.23 | higne4(IgG1 C220del+C229S) |
| | Second chain | SEQ ID NO.24 | |
| BCT-5455 (aCLL1-aTIM3) | First chain | SEQ ID NO.25 | hinge1(IgG1 WT) |
| | Second chain | SEQ ID NO.26 | |
| BCT-8182 (CLL1 control) | First chain | SEQ ID NO.31 | hinge1(IgG1 WT) |
| | Second chain | SEQ ID NO.32 | |
| BCT-8384 (aTIM3 control) | First chain | SEQ ID NO.33 | hinge1(IgG1 WT) |
| | Second chain | SEQ ID NO.34 | |
| Fc(knob) | | SEQ ID NO.35 | |
| Fc(hole) | | SEQ ID NO.36 | |
| Anti-cMet | VH nucleotide sequence | SEQ ID NO.37 | |
| | VH amino acid sequence | SEQ ID NO.38 | |
| | VL nucleotide sequence | SEQ ID NO.39 | |
| | VL amino acid sequence | SEQ ID NO.40 | |
| Anti-EGFR (panitumumab) | VH nucleotide sequence | SEQ ID NO.41 | |
| | VH amino acid sequence | SEQ ID NO.42 | |
| | VL nucleotide sequence | SEQ ID NO.43 | |
| | VL amino acid sequence | SEQ ID NO.44 | |
| Anti-EGFR (cetuximab) | VH nucleotide sequence | SEQ ID NO.45 | |
| | VH amino acid sequence | SEQ ID NO.46 | |
| | VL nucleotide sequence | SEQ ID NO.47 | |
| | VL amino acid sequence | SEQ ID NO.48 | |
| Anti-EGFR (necitumumab) | VH nucleotide sequence | SEQ ID NO.49 | |
| | VH amino acid sequence | SEQ ID NO.50 | |
| | VL nucleotide sequence | SEQ ID NO.51 | |
| | VL amino acid sequence | SEQ ID NO.52 | |
| Anti-cMet | CDR-H1 | SEQ ID NO.53 | SYGIS |
| | CDR-H2 | SEQ ID NO.54 | WISAYNGYTNYAQKLQG |
| | CDR-H3 | SEQ ID NO.55 | DLRGTNYFDY |
| | CDR-L1 | SEQ ID NO.56 | RASQGISNWLA |
| | CDR-L2 | SEQ ID NO.57 | AASSLLS |
| | CDR-L3 | SEQ ID NO.58 | QQANSFPIT |
| Anti-EGFR (panitumumab) | CDR-H1 | SEQ ID NO.59 | SGDYYWT |
| | CDR-H2 | SEQ ID NO.60 | HIYYSGNTNYNPSLKS |
| | CDR-H3 | SEQ ID NO.61 | DRVTGAFDI |
| | CDR-L1 | SEQ ID NO.62 | QASQDISNYLN |
| | CDR-L2 | SEQ ID NO.63 | DASNLET |
| | CDR-L3 | SEQ ID NO.64 | QHFDHLPLA |
| Anti-EGFR (cetuximab) | CDR-H1 | SEQ ID NO.65 | NYGVH |
| | CDR-H2 | SEQ ID NO.66 | VIWSGGNTDYNTPFTS |
| | CDR-H3 | SEQ ID NO.67 | ALTYYDYEFAY |
| | CDR-L1 | SEQ ID NO.68 | RASQSIGTNIH |
| | CDR-L2 | SEQ ID NO.69 | YASESIS |
| | CDR-L3 | SEQ ID NO.70 | QQNNNWPTT |
| Anti-EGFR (necitumumab) | CDR-H1 | SEQ ID NO.71 | SGDYYWS |
| | CDR-H2 | SEQ ID NO.72 | YIYYSGSTDYNPSLKS |
| | CDR-H3 | SEQ ID NO.73 | VSIFGVGTFDY |
| | CDR-L1 | SEQ ID NO.74 | RASQSVSSYLA |
| | CDR-L2 | SEQ ID NO.75 | DASNRAT |
| | CDR-L3 | SEQ ID NO.76 | HQYGSTPLT |
| BEC-2728(acMET-EGFR) | First chain | SEQ ID NO.77 | hinge1(IgG1 WT) |
| | Second chain | SEQ ID NO.78 | |
| BEC-2930(acMET-EGFR) | First chain | SEQ ID NO.79 | hinge5(IgG1 C226del) |
| | Second chain | SEQ ID NO.80 | |
| BEC-3132(acMET-EGFR) | First chain | SEQ ID NO.81 | hinge6(IgG1 C229del) |
| | Second chain | SEQ ID NO.82 | |
| BEC-3334(acMET-EGFR) | First chain | SEQ ID NO.83 | hinge7(IgG1 C220del+C226del) |
| | Second chain | SEQ ID NO.84 | |
| BEC-3536(acMET-EGFR) | First chain | SEQ ID NO.85 | hinge8(IgG1 C220del+C229del) |
| | Second chain | SEQ ID NO.86 | |
| BEC-3738(acMET-EGFR) | First chain | SEQ ID NO.87 | hinge9(IgG1 C226del+C229del) |
| | Second chain | SEQ ID NO.88 | |
| BEC-3940(acMET-EGFR) | First chain | SEQ ID NO.89 | hinge10(IgG1 C220S) |
| | Second chain | SEQ ID NO.90 | |
| BEC-4142(acMET-EGFR) | First chain | SEQ ID NO.91 | hinge11(IgG1 C226S) |
| | Second chain | SEQ ID NO.92 | |
| BEC-4344(acMET-EGFR) | First chain | SEQ ID NO.93 | hinge12(IgG1 C229S) |
| | Second chain | SEQ ID NO.94 | |
| BEC-4546(acMET-EGFR) | First chain | SEQ ID NO.95 | hinge13(IgG1 C226S+C229S) |
| | Second chain | SEQ ID NO.96 | |
| MTE-6263 | First chain | SEQ ID NO.97 | hinge1(IgG1 WT) |
| | Second chain | SEQ ID NO.98 | |
| MTE-6465 | First chain | SEQ ID NO.99 | hinge2(IgG1 C220del) |
| | Second chain | SEQ ID NO.100 | |
| MTE-6667 | First chain | SEQ ID NO.101 | hinge5(IgG1 C226del) |
| | Second chain | SEQ ID NO.102 | |
| MTE-6869 | First chain | SEQ ID NO.103 | hinge6(IgG1 C229del) |
| | Second chain | SEQ ID NO.104 | |
| MTE-7071 | First chain | SEQ ID NO.105 | hinge10(IgG1 C220S) |
| | Second chain | SEQ ID NO.106 | |
| MTE-7273 | First chain | SEQ ID NO.107 | hinge11(IgG1 C226S) |
| | Second chain | SEQ ID NO.108 | |
| MTE-7475 | First chain | SEQ ID NO.109 | hinge12(IgG1 C229S) |
| | Second chain | SEQ ID NO.110 | |
| MTE-7677 | First chain | SEQ ID NO.111 | hinge3(IgG1 C220del+C226S) |
| | Second chain | SEQ ID NO.112 | |
| MTE-7879 | First chain | SEQ ID NO.113 | hinge4(IgG1 C220del+C229S) |
| | Second chain | SEQ ID NO.114 | |
| MTE-8081 | First chain | SEQ ID NO.115 | hinge13(IgG1 C226S+C229S) |
| | Second chain | SEQ ID NO.116 | |
| BEC-9293 | First chain | SEQ ID NO.117 | hinge14(IgG4 WT) |
| | Second chain | SEQ ID NO.118 | |
| BEC-9495 | First chain | SEQ ID NO.119 | hinge14(IgG4 WT) |
| | Second chain | SEQ ID NO.120 | |
| BEC-9697 | First chain | SEQ ID NO.121 | hinge15(IgG4 C226S) |
| | Second chain | SEQ ID NO.122 | |
| BEC-9898 | First chain | SEQ ID NO.123 | hinge16(IgG4 C229S) |
| | Second chain | SEQ ID NO.124 | |
| BEC-0001 | First chain | SEQ ID NO.125 | hinge15(IgG4 C226S) |
| | Second chain | SEQ ID NO.126 | |
| BEC-0203 | First chain | SEQ ID NO.127 | hinge16(IgG4 C229S) |
| | Second chain | SEQ ID NO.128 | |
| BEC-0405 | First chain | SEQ ID NO.129 | hinge17(IgG4C226S+C229S) |
| | Second chain | SEQ ID NO.130 | |
| BEC-0607 | First chain | SEQ ID NO.131 | hinge14(IgG4 WT) |
| | Second chain | SEQ ID NO.132 | |
| BEC-0809 | First chain | SEQ ID NO.133 | hinge18(IgG4 C226del) |
| | Second chain | SEQ ID NO.134 | |
| BEC-1011 | First chain | SEQ ID NO.135 | hinge19(IgG4 C229del) |
| | Second chain | SEQ ID NO.136 | |
| hinge1(IgG1 WT) | | SEQ ID NO.137 | EPKSCDKTHTCPPCP |
| hinge2(IgG1 C220del) | | SEQ ID NO.138 | EPKSDKTHTCPPCP |
| hinge3(IgG1 C220del+C226S) | | SEQ ID NO.139 | EPKSDKTHTSPPCP |
| hinge4(IgG1 C220del+C229S) | | SEQ ID NO.140 | EPKSDKTHTCPPSP |
| hinge5(IgG1 C226del) | | SEQ ID NO.141 | EPKSCDKTHTPPCP |
| hinge6(IgG1 C229del) | | SEQ ID NO.142 | EPKSCDKTHTCPPP |
| hinge7(IgG1 C220del+C226del) | | SEQ ID NO.143 | EPKSDKTHTPPCP |
| hinge8(IgG1 C220del+C229del) | | SEQ ID NO.144 | EPKSDKTHTCPPP |
| hinge9(IgG1 C226del+C229del) | | SEQ ID NO.145 | EPKSCDKTHTPPP |
| hinge10(IgG1 C220S) | | SEQ ID NO.146 | EPKSSDKTHTCPPCP |
| hinge11(IgG1 C226S) | | SEQ ID NO.147 | EPKSCDKTHTSPPCP |
| hinge12(IgG1 C229S) | | SEQ ID NO.148 | EPKSCDKTHTCPPSP |
| hinge13(IgG1 C226S+C229S) | | SEQ ID NO.149 | EPKSCDKTHTSPPSP |
| hinge14(IgG4 WT) | | SEQ ID NO.150 | ESKYGPPCPPCP |
| hinge15(IgG4 C226S) | | SEQ ID NO.151 | ESKYGPPSPPCP |
| hinge16(IgG4 C229S) | | SEQ ID NO.152 | ESKYGPPCPPSP |
| hinge17(IgG4C226S+ C229S) | | SEQ ID NO.153 | ESKYGPPSPPSP |
| hinge18(IgG4 C226del): | | SEQ ID NO.154 | ESKYGPPPPCP |
| hinge19(IgG4 C229d) | | SEQ ID NO.155 | ESKYGPPCPPP |
| hinge20(IgG2 WT) | | SEQ ID NO.156 | ERKCCVECPPCP |
| hinge21(IgG2 C226S) | | SEQ ID NO.157 | ERKCCVESPPCP |
| hinge22(IgG2 C229S) | | SEQ ID NO.158 | ERKCCVECPPSP |
| hinge23(IgG2 C220S) | | SEQ ID NO.159 | ERKCSVECPPCP |
| hinge24(IgG2 C219S) | | SEQ ID NO.160 | ERKSCVECPPCP |
| hinge25(IgG2 C226S+C229S) | | SEQ ID NO.161 | ERKCCVESPPSP |
| hinge26(IgG2 C219S+C220S) | | SEQ ID NO.162 | ERKSSVECPPCP |
| hinge27(IgG2 C219S+C220S+C22 6S) | | SEQ ID NO.163 | ERKSSVESPPCP |
| hinge28(IgG2 C219S+C220S+C22 9S) | | SEQ ID NO.164 | ERKSSVEZCPPSP |
| hinge29(IgG3 WT) | | SEQ ID NO.165 | ELKTPLGDTTHTCPRCPEPKSC DTPPPCPRCPEPKSCDTPPPCPR CPEPKSCDTPPPCPRCP |
| hinge30(IgG1 C226S+C229del) | | SEQ ID NO.166 | EPKSCDKTHTSPPP |
| hinge31(IgG1 C226del+C229S) | | SEQ ID NO.167 | EPKSCDKTHTPPSP |
| hinge32(IgG1 C220S+C229S) | | SEQ ID NO.168 | EPKSSDKTHTCPPSP |
| hinge33(IgG1 C220S+C226S) | | SEQ ID NO.169 | EPKSSDKTHTSPPCP |
| hinge34(IgG1 C220S+C229del) | | SEQ ID NO.170 | EPKSSDKTHTCPPP |
| hinge35(IgG1 C220S+C226del) | | SEQ ID NO.171 | EPKSSDKTHTPPCP |
| BEC-0102 | First chain | SEQ ID NO.172 | hinge2(IgG1 C220del) |
| | Second chain | SEQ ID NO.173 | |
| BEC-0304 | First chain | SEQ ID NO.174 | hinge2(IgG1 C220del) |
| | Second chain | SEQ ID NO.175 | hinge2(IgG1 C220del) |
| BPB-0506 | First chain | SEQ ID NO.176 | hinge2(IgG1 C220del) |
| | Second chain | SEQ ID NO.177 | hinge2(IgG1 C220del) |
| BPB-0708 | First chain | SEQ ID NO.178 | hinge2(IgG1 C220del) |
| | Second chain | SEQ ID NO.179 | |
| MTE-8687 | First chain | SEQ ID NO.180 | hinge2(IgG1 C220del) |
| | Second chain | SEQ ID NO.181 | |
| MTE-9091 | First chain | SEQ ID NO.182 | hinge2(IgG1 C220del) |
| | Second chain | SEQ ID NO.183 | |

### Example 2. Preparation of Antibodies and Their Conjugates 2.1 Expression and Purification of Antibodies

The eukaryotic expression vector plasmids constructed in Example 1 (see Table 1) were co-transfected into FreeStyle HEK293 cells at a cell density of 2.5×10⁶ cells/mL, and cultured at 125 rpm, 37°Cand 5% CO2 for 5-6 days. The cell-culture supernatant was collected by centrifugation and filtered through a 0.22 µm filter. The fusion antibodies were purified using Protein A Resin (GenScript) according to the manufacturer's instructions. Their concentrations were determined by A280 and BCA assay (Pierce). The Protein A resin-purified fusion antibodies were further separated, purified and buffer-exchanged using a GE ÄKTA chromatography system and a Superdex 200 Increase 10/300 GL size-exclusion chromatography column in PBS buffer (pH 7.4). The purified samples were stored in PBS buffer (pH 7.4). The composition and purity of the antibodies were examined by SDS-PAGE under reducing and non-reducing conditions. The monomeric fraction of the antibodies in a saline environment was analyzed by size-exclusion chromatography.

### 2.2 Preparation of Antibody Conjugates

In this study, compounds bearing a maleimide linker based on two toxin molecules, MMAE and Dxd, were selected for the preparation of conjugates. A certain amount of the antibody obtained in Example 2.1 was subjected to an optimized reduction reaction using tris(2-carboxyethyl)phosphine hydrochloride (TCEP; CAS No. 51805-45-9), so that the interchain disulfide bonds between the first and second chains of the antibody were opened. A payload bearing a maleimide linker (Deruxtecan (Dxd) (CAS No. 1599440-13-7) or vc-MMAE (CAS No. 646502-53-6) was then added, and alkylation occurred through nucleophilic Michael addition of thiol groups to form stable thioether bonds, thereby producing relatively homogeneous conjugated products. For the reduction reaction, the antibody and TCEP were reacted at a molar ratio of 1:20 in a 37°C incubator for 60 min. After completion, the sample was removed and cooled on ice, and a pre-cooled linker-bearing payload was then added at a payload-to-antibody molar ratio of 20:1 and reacted on ice for 2 h. TCEP introduced during reduction and excess unreacted payload were removed using a 40 kDa Zeba desalting spin column (CAS: 87766), while the conjugated sample was exchanged from the preceding DPBS buffer system into a suitable buffer system containing 10 mg/mL sucrose, 20 mg/mL glycine and 1.47 mg/mL glutamic acid at pH 4.0. The composition and purity of the conjugated products were examined by SDS-PAGE under reducing and non-reducing conditions, and the antibody conjugates were analyzed by size-exclusion chromatography.

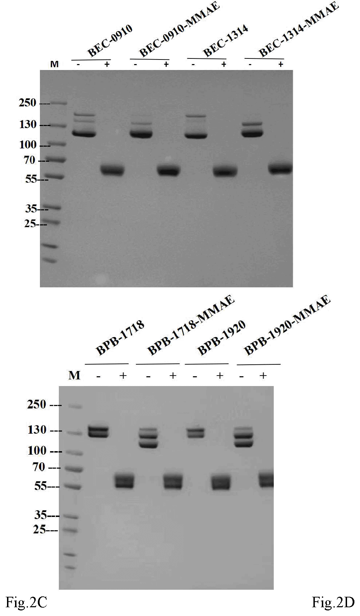

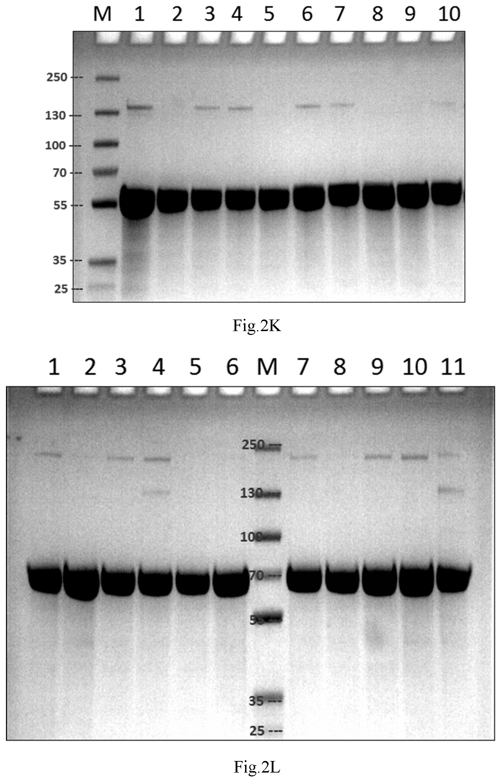

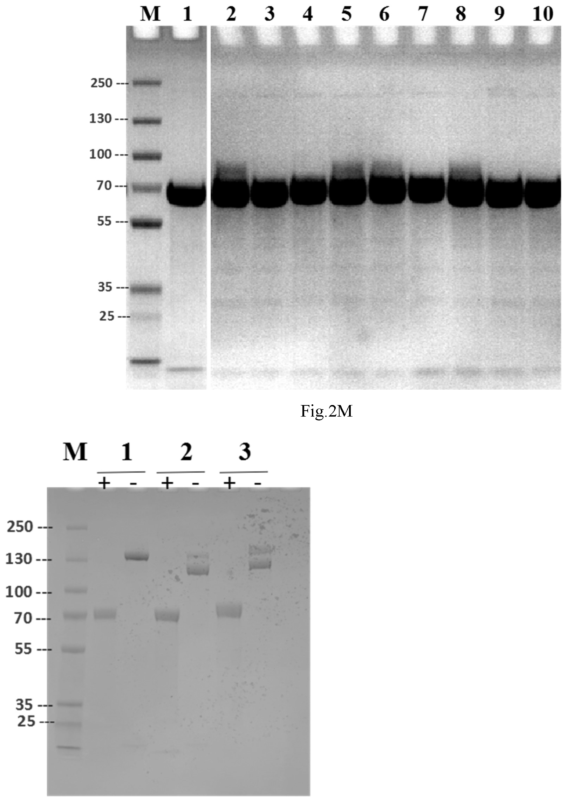

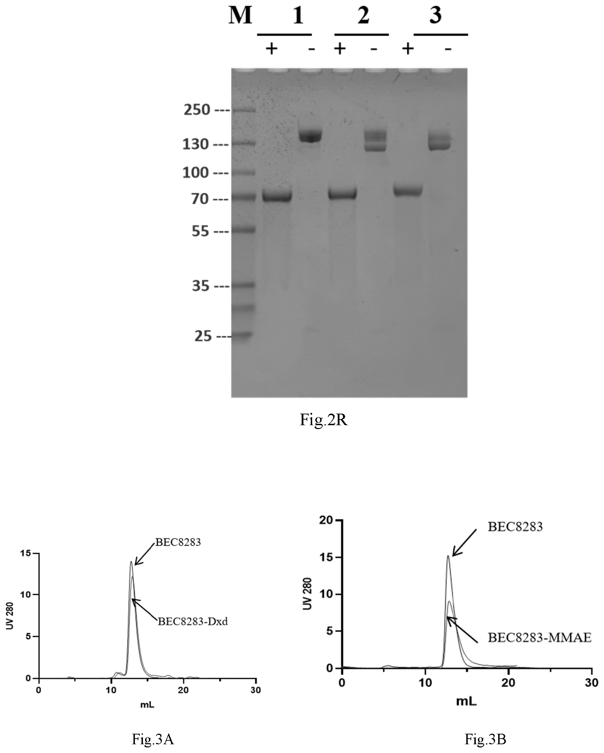

The results are shown in Figures 2 and 3. Under non-reducing SDS-PAGE conditions, the bispecific antibodies and their conjugates had almost no or only very small amount of free first or second chains, indicating that the products obtained after conjugation of the antibodies with the payload had structural stability similar to that of the unconjugated antibodies. In contrast, after conjugation of a payload to the hinge disulfide bonds of the control monoclonal antibodies aEGFR mAb and aHER2 mAb, clearly detectable free light chains and heavy chains were observed under non-reducing SDS conditions.

### Example 3. Thermodynamic Stability Testing

The conjugated and unconjugated antibodies of Example 2, each at a concentration of 1 mg/mL, were mixed with freshly prepared thermal shift dye and shift buffer (Protein Thermal Shift^{™} Dye Kit, Thermo Fisher Scientific, Cat. No. 4461146) at the ratios recommended by the manufacturer. Thermal scanning was performed from 25°C to 99°C using a ViiA^{™} 7 Real-Time PCR System at a heating rate of 0.05°C/s. The melting temperature (Tm) values were calculated using the "Area under curve (AUC)" analysis model in GraphPad Prism 7 software. Atezolizumab (GenScript) was used as a monoclonal antibody control . Each group was tested in duplicate to ensure reproducibility. The results are shown in Table 2-1. The melting temperatures (Tm) of the bispecific antibodies were close to the Tm value of the control antibody Atezolizumab. The Tm values of the Dxd- or MMAE-conjugated bispecific antibodies were close to those of the corresponding unconjugated bispecific antibodies, indicating that both the bispecific antibodies and their conjugates had thermal stability similar to that of the monoclonal antibody Atezolizumab.

**Table 2-1: Tm Values of Antibodies and Their Conjugates**

| Sample | Tm1 | Tm2 | Tm3 |
|---|---|---|---|
| Atezolizumab | 60.79°C | 84.98°C | -- |
| BCT-5455 | 67.65°C | 72.69°C | 82.19°C |
| BCT-5455 plus TCEP treatment | 67.46°C | 72.31°C | -- |
| BCT-5455-Dxd plus TCEP treatment | 60.68°C | 71.34°C | -- |
| BPB-7172 | 70.08°C | 80.33°C | -- |
| BPB-7172 plus TCEP treatment | 70.27°C | 80.72°C | -- |
| BPB-7172-Dxd | 63.50°C | 71.24°C | -- |
| BPB-7172-MMAE | 58.08°C | 63.59°C | -- |
| BPB-7374 | 70.82°C | 83.00°C | -- |
| BPB-6734 | 66.68°C | 80.39°C | -- |
| BPB-6734-MMAE | 68.61°C | 81.16°C | -- |
| BPB-6734-Dxd | 65.20°C | 87.42°C | -- |
| BEC-8283 | 62.92°C | 81.11°C | -- |
| BEC-8283-Dxd | 61.58°C | 71.26°C | -- |
| BEC-8283-MMAE | 61.58°C | 79.38°C | -- |
| BEC-8485 | 61.55°C | 80.69°C | -- |
| BEC-8485-Dxd | 61.74°C | 79.34°C | -- |
| BEC-8485-MMAE | 62.52°C | 80.11°C | -- |
| BEC-6364 | 70.08°C | 74.72°C | 83.04°C |
| BEC-0910 | 67.99°C | 83.34°C | |
| BEC-0910-MMAE | 60.86°C | 81.27°C | |
| BEC-1314 | 61.26°C | 83.85°C | |
| BEC-1314-MMAE | 67.99°C | 83.34°C | |
| BEC0809 | 57.33 | | |
| BEC1011 | 58.72 | | |

**Table 2-2: Tm and Tagg Values of Antibodies**

| Sample | Tagg (°C) | Tm-1 (°C) | Tm-2 (°C) |
|---|---|---|---|
| BEC2728 | 71.3 | 71.1 | 76.5 |
| BEC8283 | 71.1 | 70.8 | 76.7 |
| BEC2930 | 57.7 | 71.1 | 76.6 |
| BEC3132 | 56.4 | 70.5 | 76.7 |
| BEC3334 | 56.6 | 70.8 | 76.7 |
| BEC3536 | 57.7 | 70.5 | 76.6 |
| BEC3738 | 57.9 | 69.4 | 76.7 |
| BEC3940 | 55.8 | 70.7 | 76.6 |
| BEC4142 | 57.4 | 71.1 | 76.6 |
| BEC4344 | 57.2 | 69.7 | 76.4 |
| BEC4546 | 57.0 | 67.3 | 76.3 |

**Table 2-3: Tm Values of Antibodies**

| Sample | Tm-1 (°C) | Tm-2 (°C) | Tm-3 (°C) |
|---|---|---|---|
| MTE6263 | 68.83 | 74.39 | -- |
| MTE6465 | 69.03 | 74.19 | -- |
| MTE6667 | 63.28 | 67.64 | 74.19 |
| MTE6869 | 62.68 | 67.25 | 74.19 |
| MTE7071 | 62.68 | 67.84 | 74.59 |
| MTE7273 | 68.64 | 74.59 | -- |
| MTE7475 | 61.69 | 74.19 | -- |
| MTE7677 | 62.88 | 74.59 | -- |
| MTE7879 | 62.49 | 66.65 | 73.79 |
| MTE8081 | 65.46 | 74.39 | -- |

**Table 2-4: Tm Values of Fusion Antibodies**

| Sample | Tm-1 (°C) |
|---|---|
| BEC9293 | 64.07 |
| BEC9495 | 64.67 |
| BEC9697 | 63.48 |
| BEC9899 | 63.28 |
| BEC0001 | 63.68 |
| BEC0203 | 63.28 |
| BEC0405 | 63.28 |
| BEC0607 | 65.26 |
| BEC0809 | 57.33 |
| BEC1011 | 58.72 |

### Example 4. Mass Spectrometry Analysis

The bispecific antibodies and conjugates of Example 2, each at a concentration of 1 mg/mL, were incubated with PNGase F (NEB) overnight at 37°C. The deglycosylated samples were reduced by addition of 10 mM DTT. The reduced samples were injected onto a 300SB-C8 column (2.1×50 mm) of an HPLC-Q-TOF-MS system (Agilent, USA) for mass spectrometric analysis. DAR values were calculated using the "Area Under Curve (AUC)" analysis in GraphPad Prism 7 software. The results are shown in FIG. 4. The molecular weights of the bispecific antibodies and their conjugates were substantially consistent with the theoretically predicted values. Both chains of the Dxd- or MMAE-conjugated bispecific antibodies carried a specific number of small-molecule drugs, and the number was the same as the number of Cys residues reduced in the linker peptides, with the DAR being a fixed value.

### Example 5. Antigen Binding Activity Assay

A target antigen was coated onto a 96-well ELISA plate at 100 ng/100 µL/well and incubated overnight at 4°C. After blocking at room temperature for 1 hour with PBST containing 3% skim milk powder (0.5% Tween-20 in PBS), the plate was washed with PBST. The bispecific antibodies and corresponding conjugates were diluted with blocking buffer to 50 nM as the starting concentration and then serially diluted five-fold to provide a total of eight concentrations. One hundred microliters of each diluted bispecific antibody, corresponding conjugate, or control antibody sample was added to each well and incubated at room temperature for 1 hour. After three washes with PBST, 100 µL of HRP-labeled goat anti-human Fc monoclonal antibody diluted 1:500 according to the instructions was added to each well and incubated at room temperature for 1 hour. The plate was then washed five times with PBST. One hundred microliters of freshly prepared TMB chromogenic reagent (BioLegend, Cat. No. 421101) was added to each washed well and incubated for 10 minutes at room temperature in the dark. Then, 100 µL of 1 M H2SO4 was added to each well. After incubation for 2 minutes, absorbance was read at 450 nm using a microplate reader. EC50 values were calculated using the 'log(agonist) vs. response - Variable slope (four parameters)' analysis model in GraphPad Prism 7 software. Each group was tested in duplicate to ensure reproducibility.

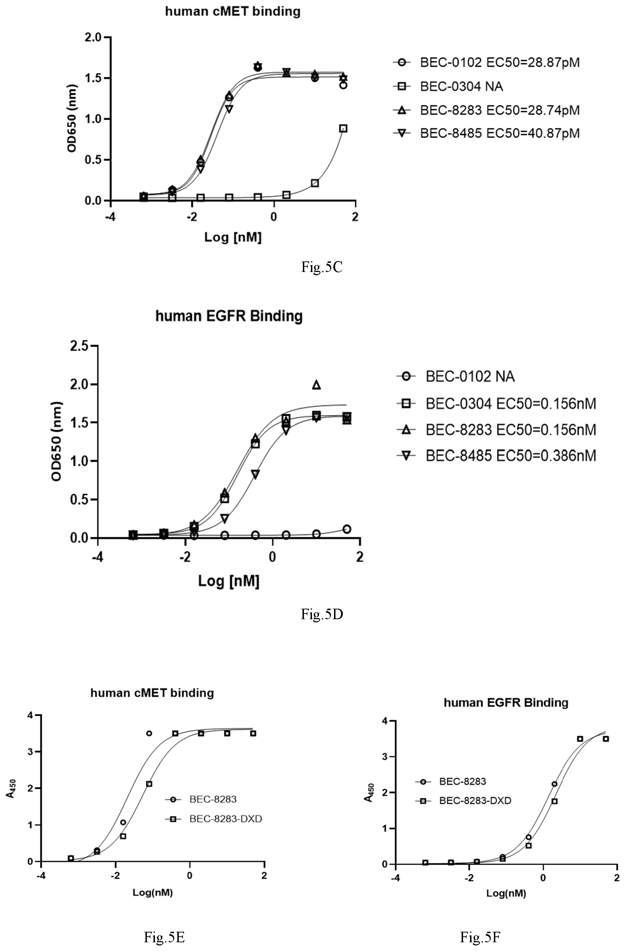

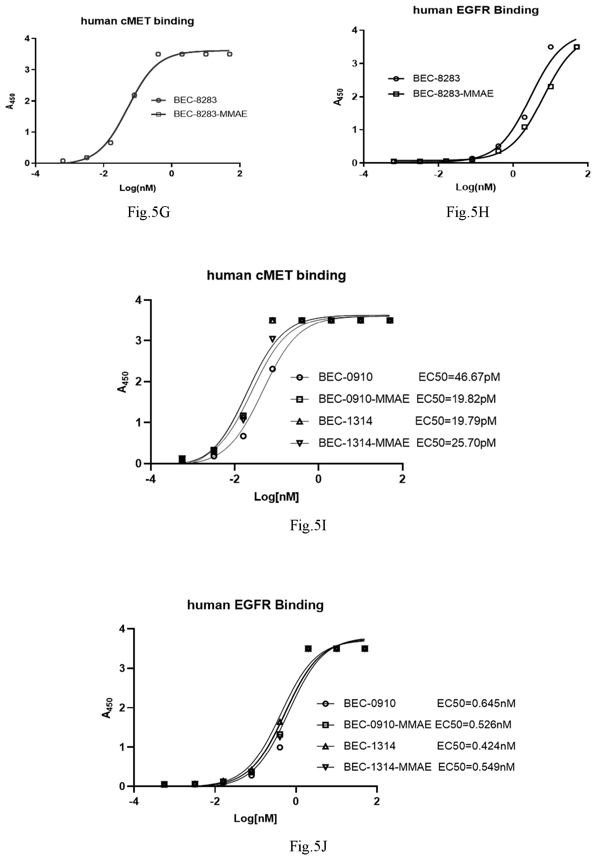

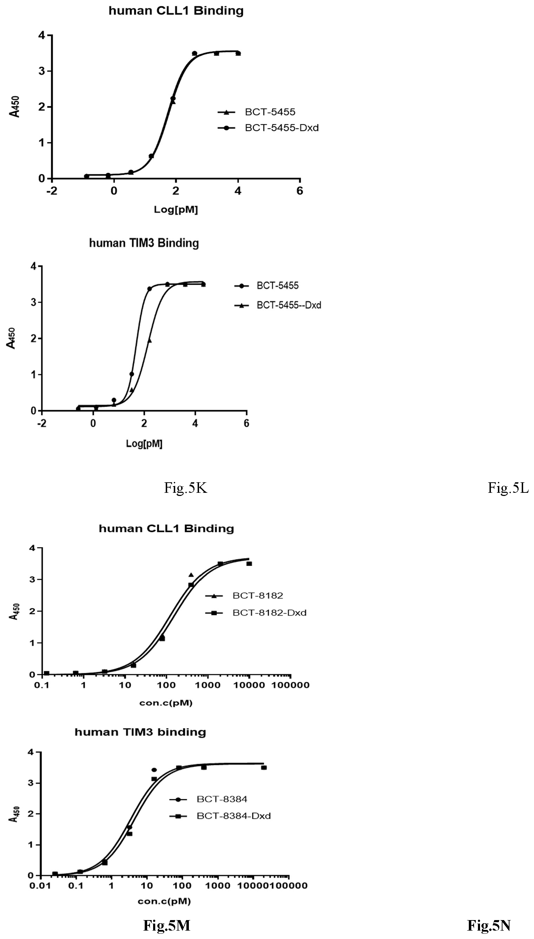

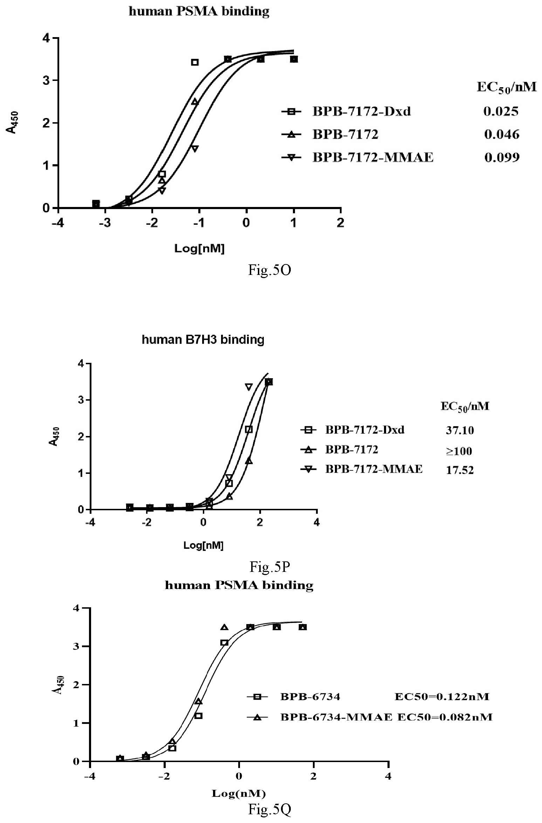

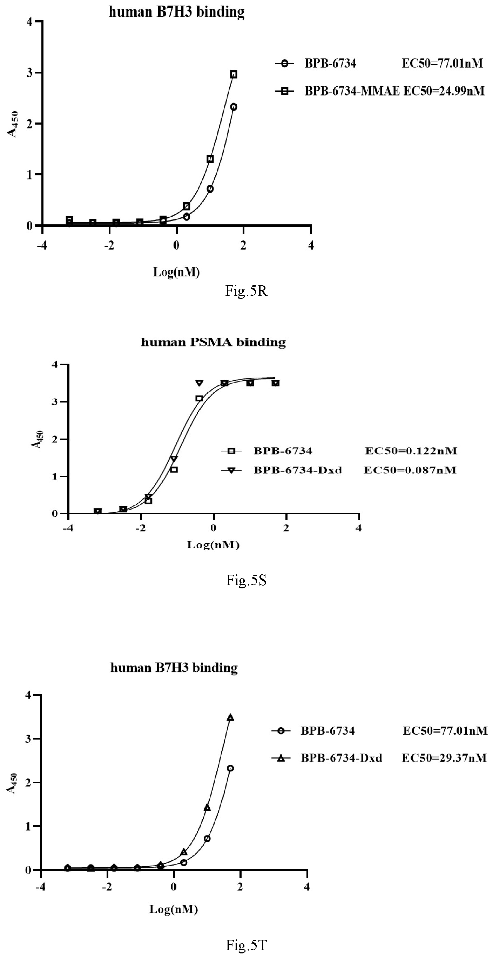

The results are shown in FIG. 5. The binding activities of the Dxd- or MMAE-based bispecific antibody conjugates toward the two target antigens showed no significant difference from those of the unconjugated bispecific antibodies, indicating that conjugation of Dxd or MMAE to Cys residues in the first and second linker peptides of the antibody did not affect the antigen-binding activity of the antibody moiety.

### Example 6. Cell Surface Receptor Binding Activity Assay

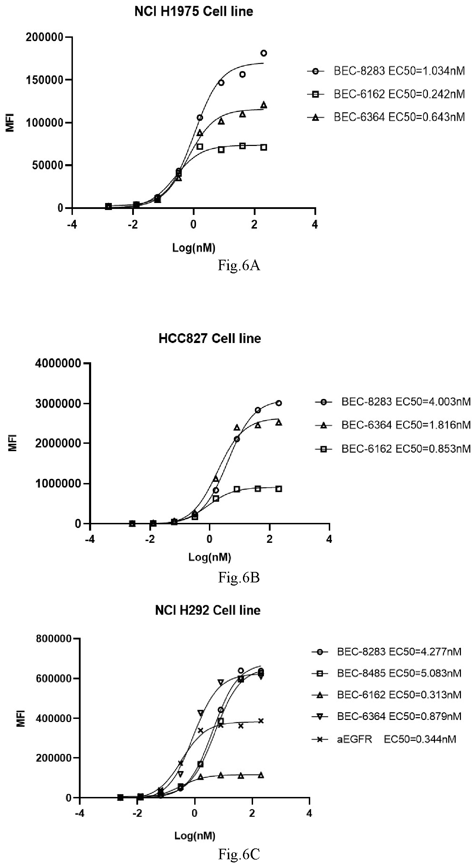

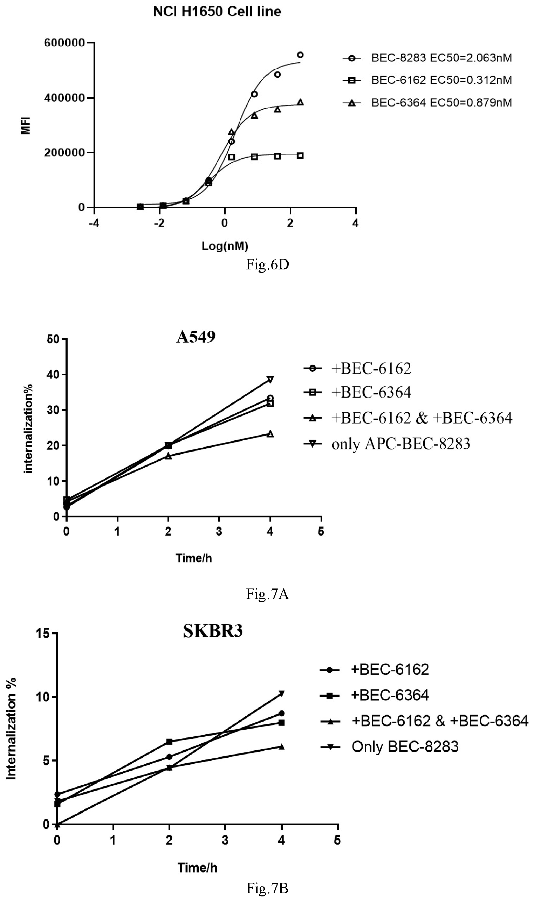

Cell lines having antigens targeted by the antibodies were cultured adherently in DMEM containing 10% FBS. After trypsin digestion, 2×10⁵ cells were dispensed into each flow-cytometry staining well and blocked with pre-cooled 2% FBS-PBS blocking solution for 30 minutes. The bispecific antibodies and the corresponding control-antibody samples were diluted with the blocking solution to 200 nM, which was used as the starting concentration, and were subjected to five-fold serial dilution to provide a total of eight concentrations. A concentration of 0 nM was included as the staining background. One hundred microliters of each diluted antibody sample was added to each well and incubated at 4°C for 30 minutes. After washing three times with PBS, 100 µL of APC-labeled anti-human Fc monoclonal antibody diluted according to the manufacturer's instructions was added to each well and incubated at 4°C for 30 minutes, followed by three washes with PBS. Two hundred microliters of blocking solution was added to each washed flow-cytometry tube, and the resuspended cell suspension was used for flow-cytometric analysis. EC50 values were calculated using the "log(agonist) vs. response -- Variable slope (four parameters)" analysis model in GraphPad Prism 7. Each group of data was tested in duplicate to ensure reproducibility. The results are shown in Table 3 and Figure 6. The bispecific antibodies targeting EGFR and cMet exhibited a synergistic effect in binding to the two targets. The ratios of the abundance of the two target antigens on the surfaces of the respective cells are shown in Table 4.

**Table 3: EC₅₀ of Bispecific Antibody Binding to Cell Surface Antigens**

| Protein # | EC50 (nM) | | | | | | | |
|---|---|---|---|---|---|---|---|---|
| | A549 | A431 | MDA231 | HEPG2 | C42 | SKBR3 | U87MG | MCF7 |
| BEC-6162 | 0.4 | 0.2 | 0.4 | 0.2 | 0.2 | 0.2 | 0.3 | 0.3 |
| BEC-6364 | 1 | 2 | 1 | 0.2 | 0.3 | 0.3 | 0.7 | 0.4 |
| BEC-8283 | 3.5 | 6 | 5.5 | 0.5 | 1.8 | 1.1 | 3.6 | 1.9 |
| BEC-8485 | 4 | 5.7 | 6.7 | 0.4 | 2.6 | 1.3 | 4.3 | 2.9 |

**Table 4: Abundance of Dual Target Antigens on Cell Surfaces**

| Cell line | cMET | RelativecMET density | EGFR | EGFR-MFI/BLK | EGFR/cMET |
|---|---|---|---|---|---|
| A549 | High | 1.75×10^5 | Med | 8.16×10^5 | 5 |
| A431 | High | 1.54×10^5 | High | 74.22×10^5 | 63 |
| MDA-MB-231 | High | 1.44×10^5 | Med | 7.86×10^5 | 6 |
| HEPG2 | Med | 0.61×10^5 | Low | 0.11×10^5 | 0.25 |
| C42 | Low | 0.04×10^5 | Med | 2.37×10^5 | 53 |
| SKBR3 | Med | 0.36×10^5 | Low | 0.54×10^5 | 1.6 |
| U87MG | Med | 0.63×10^4 | Med | 2.58×10^5 | 4 |
| MCF7 | Low | 0.03×10^5 | Low | 0.07×10^5 | 2 |
| K562 | Negative | Negative | Negative | Negative | N.A. |
| NCI-H1975 | Med | 0.71×10^5 | Med | 1.21×10^5 | 1.7 |
| HCC827 | High | 8.73×10^5 | High | 25.30×10^5 | 2.8 |
| NCI-H292 | High | 1.14×10^5 | Med | 6.10×10^5 | 5.3 |
| NCI-H1650 | High | 1.89×10^5 | Med | 3.85×10^5 | 2.0 |

### Example 7. Bispecific Antibody-Mediated Internalization

The A549 and SKBR3 cell lines, which exhibited relatively large differences in the abundance of the two target antigens in Example 6, were selected. The cells were harvested after trypsin digestion and resuspended in complete DMEM containing 10% FBS. A total of 2 × 10^5 cells was dispensed into each flow-cytometry staining well and blocked with pre-cooled 2% FBS-PBS blocking solution for 30 minutes. The control antibodies BEC-6162 and BEC-6364 of Example 2 were each diluted to 1 µM and separately added to the flow-cytometry staining wells to block the target antigens, followed by incubation at 4°C for 1 hour. After washing three times with PBS, labeled APC-YF-382+383 sample diluted to 50 nM was added to the washed flow-cytometry tubes and incubated at 4°C for 30 minutes. After one wash with PBS, the flow-cytometry staining tubes were placed in a 37°C incubator to allow internalization. Corresponding samples were removed at different time points and washed once with PBS. Each sample was divided into two portions. One portion was treated with acidic buffer (0.2 M glycine, 0.15 M NaCl, pH 2.0) at 4°C for 10 minutes, and the other portion was treated with 2% FBS-PBS blocking solution at 4°C for the same period. After one wash with PBS, 30 µL of 4% paraformaldehyde fixative was added to each tube and incubated at room temperature for 20 minutes. After one further wash with PBS, 200 µL of blocking solution was added to each washed tube, and the resuspended cell suspension was subjected to flow-cytometric analysis. The internalization results were analyzed using the "XY Table" function in GraphPad Prism 7. Each group of data was tested in duplicate to ensure reproducibility.

The results are shown in Figure 7. In A549 cells, after blocking the cMet and EGFR target antigens separately, differences in APC-BEC-8283-mediated internalization relative to the unblocked group appeared at 2 hours and were greatest at 4 hours. When both cMet and EGFR were blocked, APC-BEC-8283-mediated internalization was significantly reduced and was lower than that observed in either single-antigen blocking group. Similar results were observed in SKBR3 cells, suggesting that both the externally positioned anti-cMet antibody and the internally positioned anti-EGFR antibody of the bispecific antibody can undergo favorable internalization.

### Example 8. Bispecific Antibody-Mediated Internalization

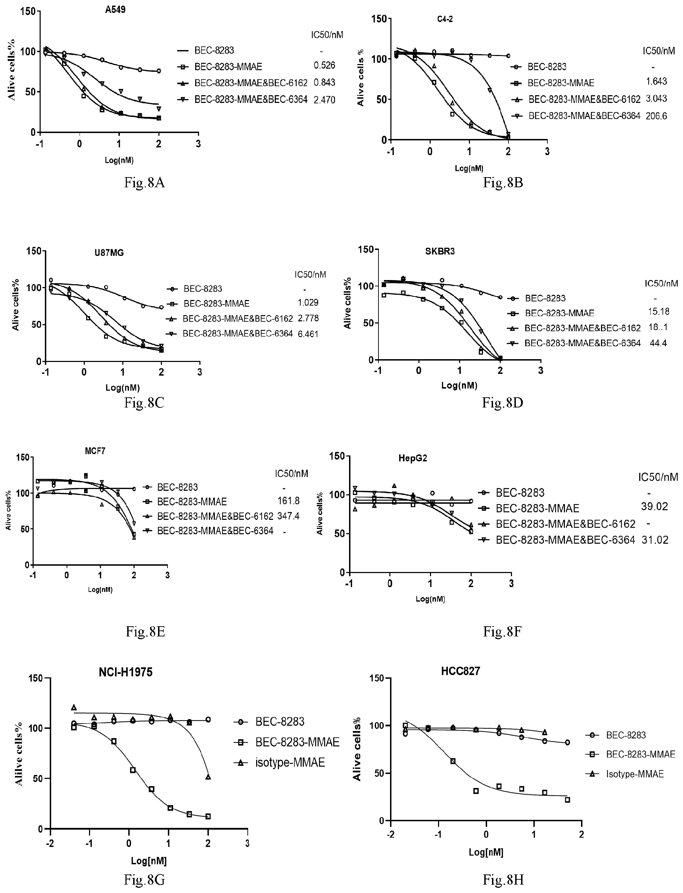

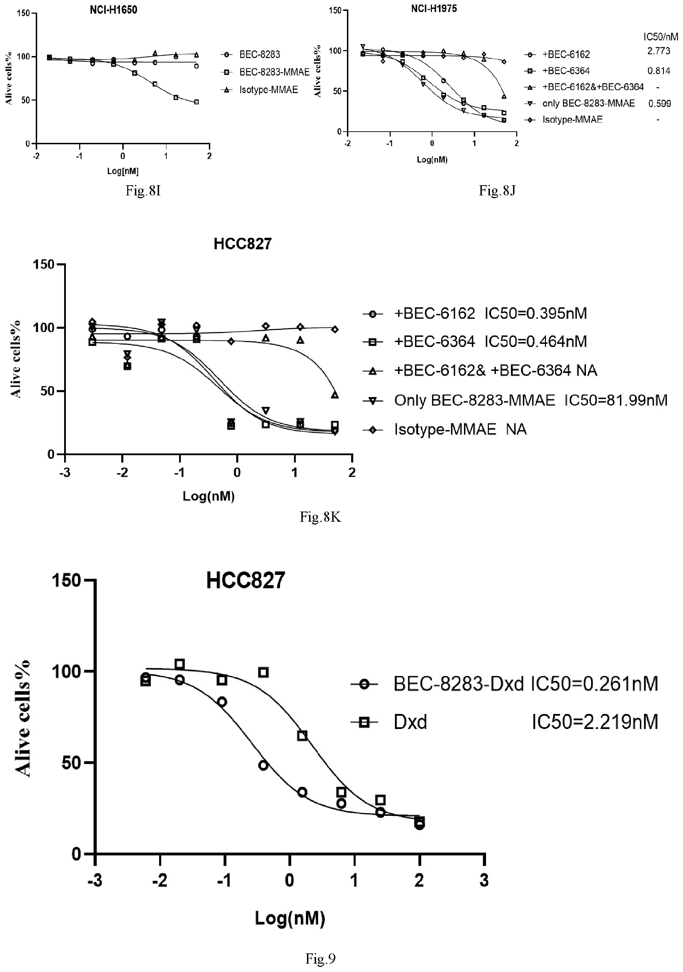

Cell lines having antigens targeted by the antibodies on their surfaces were cultured. A certain amount of the above suspension cells was collected, centrifuged at 500×g, and the supernatant was discarded. The cells were resuspended in RPMI 1640 complete medium containing 10% FBS to a density of 1×10^4 cells/mL. Using an eight-channel pipette, 100 µL was dispensed into each well of a black 96-well plate with a transparent bottom CAS:060096), and the cells were cultured in a cell incubator containing 5% CO2. A 10× EGFR monoclonal-antibody stock solution (10 µM) and a 10× cMet monoclonal-antibody stock solution (10 µM) were first prepared in DPBS. For the competitive-binding groups, the EGFR monoclonal-antibody stock solution and the cMet monoclonal-antibody stock solution were added separately to a final concentration of 1 µM and pre-incubated with the cells in the plate for 5 hours. The bispecific-antibody conjugates and corresponding control-antibody conjugates were diluted in DPBS to a highest concentration of 100 nM and serially diluted three-fold. After the 5-hour pre-incubation, the diluted conjugates were added, mixed with the cells, and cultured for 5 days in a cell incubator containing 5% CO2. After 5 days, the 96-well plate was removed and an equal volume of CellTiter-Glo reagent (CellTiter-Glo^{®} Luminescent Cell Viability Assay, CAS: G7571) was added. After incubation at room temperature for 15 minutes, luminescence was measured using a microplate reader. A conjugate of the BAB05 antibody, a double-negative control antibody, was used as the control sample. IC50 values were calculated using the "log(inhibitor) vs. response -- Variable slope (four parameters)"analysis model in GraphPad Prism 7. Each group of data was tested in duplicate to ensure reproducibility. The results are shown in Figure 8 and Table 5. The MMAE-based conjugate of the bispecific antibody BEC-8283 showed favorable cytotoxic activity against antigen-positive cells, particularly double-positive cells. Blocking either cMet or EGFR reduced the cytotoxicity of the bispecific-antibody conjugate, and blocking EGFR caused a particularly marked reduction, suggesting that the EGFR-binding arm plays a dominant role in the cytotoxic activity of the bispecific-antibody conjugate.

**Table 5: Specific Cytotoxicity of MMAE-Based Bispecific Antibody Conjugates Against Cells**

| Cell Line | Relative cMET density | Relative EGFR density | EGFR/c MET | BEC-8283-MMAE IC50/nM | MMAE IC50/nM | BEC-8283-MMAE/MMAE Ratio |
|---|---|---|---|---|---|---|
| NCI H1975 | 7.10×10^4 | 1.21×10^5 | 1.70 | 1.417 | 6.085 | 0.232 |
| NCC827 | 8.73×10^5 | 2.53×10^6 | 2.89 | 0.134 | 5.450 | 0.024 |
| NCI H292 | 1.14×10^5 | 6.10×10^5 | 5.33 | 1.220 | 1.152 | 1.059 |
| NCI H1650 | 1.89×10^5 | 3.85×10^5 | 2.03 | 4.221 | 3.947 | 1.069 |
| A549 | 1.75×10^5 | 8.16×10^5 | 5 | 0.526 | 0.612 | 0.859 |
| A431 | 1.54×10^5 | 7.42×10^6 | 63 | 2.558 | 0.072 | 35.52 |
| MDA-MB-231 | 1.44×10^5 | 7.86×10^5 | 6 | 1.514 | 0.057 | 26.56 |
| HEPG2 | 0.61×10^5 | 0.11×10^5 | 0.25 | 39.02 | 0.242 | 161 |
| C42 | 0.40×10^4 | 2.37×10^5 | 53 | 1.643 | 0.044 | 37.34 |
| SKBR3 | 3.64×10^4 | 5.45×10^4 | 1.6 | 15.18 | 0.036 | 421 |
| U87MG | 6.33×10^4 | 2.58×10^5 | 4 | 1.029 | 0.184 | 5.59 |
| MCF7 | 0.37×10^4 | 0.73×10^4 | 2 | 161.8 | 0.155 | 1043 |
| 293T | Negative | Negative | - | > 500 | 0.038 | - |

### Example 9. Cytotoxicity of Dxd-Based Conjugates

The positive cell line HCC827 was used to evaluate target-dependent toxicity mediated by a specific antibody and the corresponding control antibody-drug conjugates. A cell line having the antibody-targeted antigen on the surface was cultured. A certain amount of the above suspended cells was collected, centrifuged at 500 × g, and the supernatant was discarded. The cells were resuspended in RPMI 1640 complete medium containing 10% FBS to a density of 2 × 10^4 cells/mL. Using an 8-channel pipette, 100 µL was dispensed into each well of a clear-bottom black 96-well plate (CAS: 060096), and the cells were cultured in a cell incubator containing 5% CO2. The bispecific-antibody conjugates and the corresponding control antibody-drug conjugates were diluted with DPBS buffer to a highest final concentration of 100 nM and subjected to three-fold serial dilution. After the cells had been cultured overnight, the conjugates were added and mixed with the cells, followed by culture for 5 days in a cell incubator containing 5% CO2. After 5 days, the 96-well plate was removed, an equal volume of CellTiter-Glo^{®} Luminescent Cell Viability Assay reagent (CAS: G7571) was added, and the plate was incubated at room temperature for 15 minutes before measurement on a microplate reader. IC50 values were calculated using the "log(Inhibitors) vs. response -- Variable slope (four parameters)" analysis model in GraphPad Prism 7. Each group of data was tested in duplicate to ensure reproducibility. As shown in Figure 9, the DXD-based bispecific antibody BEC-8283 had a good killing effect on double-positive cells.

### Example 10. Pharmacokinetic Evaluation in Mice

C57BL/6J mice aged 6-8 weeks were administered by tail-vein injection. The bispecific antibody was administered only at a high dose of 10 mg/kg. The bispecific-antibody conjugate was administered at three doses, namely 8 mg/kg, 4 mg/kg, and 1 mg/kg. Blood was collected from the orbital sinus at different time points. The concentrations of intact bispecific antibody and bispecific-antibody conjugate in serum samples at each time point were separately detected by ELISA, and the data were processed using GraphPad Prism. The pharmacokinetic results of the bispecific antibody and its conjugate in mice are shown in Table 6. The bispecific-antibody conjugate exhibited pharmacokinetic performance similar to that of the bispecific-antibody scaffold, suggesting that the stability and integrity of the antibody moiety of the present invention were not affected after site-specific cysteine conjugation with small molecules at DAR = 6, and that the conjugate still had a good serum half-life.

**Table 6: PK Parameters of Bispecific Antibodies and Their Conjugates**

| | BsAb t1/2 | BsAb ADC t1/2 |
|---|---|---|
| BEC-8283(10mg/kg,IV) | 250.76h | -- |
| BEC-8283-MMAE(8mg/kg, IV) | 103.76h | 65.42h |
| BEC-8283-MMAE(4mg/kg, IV) | 103.76h | 68.39h |
| BEC-8283-MMAE(1mg/kg, IV) | 111.45h | 64.02h |

### Example 11: Pharmacodynamic Evaluation in Mice

### 11.1 Inhibitory Evaluation in NCI-H1975 Tumor-Bearing Mice

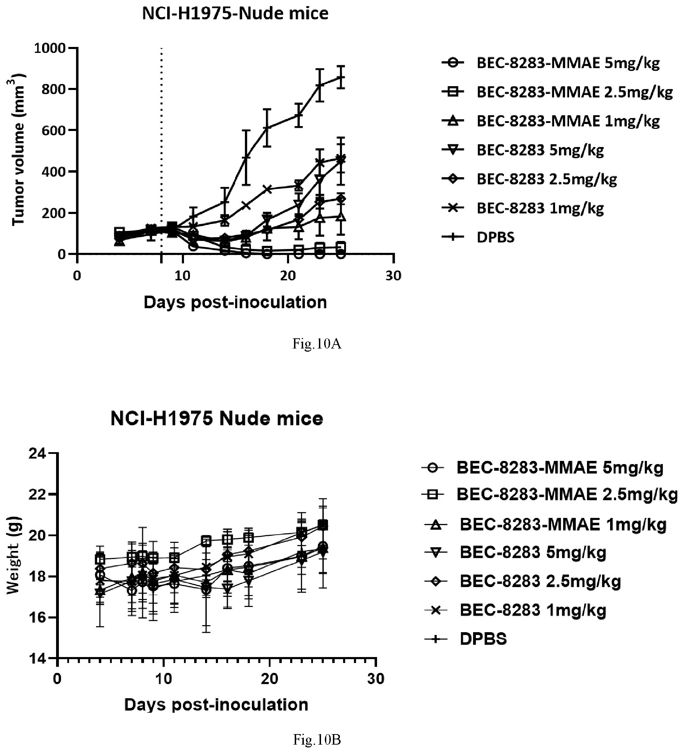

Nude mice aged 6-8 weeks were subcutaneously inoculated with tumor cells. On day 0, 5 × 10^6 NCI-H1975 cells were inoculated. On day 8, when the tumor size was about 100-200 mm3, a single dose was administered by tail-vein injection. The doses of the bispecific-antibody conjugate were 5, 2.5, and 1 mg/kg, and the corresponding doses of the bispecific antibody were 5, 2.5, and 1 mg/kg. Tumor size was monitored three times per week. As shown in Figure 10, the MMAE-based bispecific-antibody conjugate had a good inhibitory effect on NCI-H1975 tumors.

### 11.2 Inhibitory Evaluation in HCC-827 Tumor-Bearing Mice

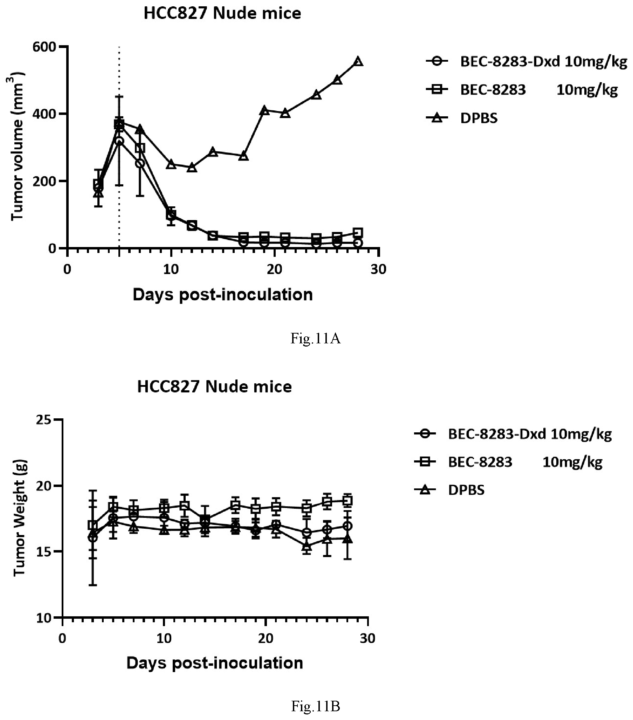

Nude mice aged 6-8 weeks were subcutaneously inoculated with tumor cells. On day 0, 5 × 10⁷6 HCC-827 cells were inoculated. On day 5, when the tumor size was about 200-500 mm3, a single dose was administered by tail-vein injection. Tumor size was monitored three times per week. As shown in Figure 11, the DXD-based bispecific-antibody conjugate effectively inhibited HCC-827 tumors.

## Claims

1. A conjugate compound comprising an antibody moiety and a heterologous moiety, wherein the antibody moiety comprises two polypeptide chains:
(a) a first polypeptide chain comprising, sequentially from N-terminus to C-terminus, binding modules A1, B1, C1, D1, and E1; and
(b) a second polypeptide chain comprising, sequentially from N-terminus to C-terminus, binding modules A2, B2, C2, D2, and E2;
wherein,
C1 binding module is a heavy chain constant region CH1;
C2 binding module is a light chain constant region CL;
D1 binding module is a first Fc;
D2 binding module is a second Fc;
B1 and/or B2 binding modules are each independently selected from a heavy chain variable region (VH), a light chain variable region (VL), a single domain antibody, a VHH domain, a receptor ligand-binding domain, a ligand receptor-binding domain, a non-immunoglobulin antigen-binding scaffold, or a single variable domain of a TCR;
A1 and/or A2 binding modules are absent or are each independently selected from a heavy chain variable region (VH), a light chain variable region (VL), a single domain antibody, a VHH domain, a receptor ligand-binding domain, a ligand receptor-binding domain, a non-immunoglobulin antigen-binding scaffold, or a single variable domain of a TCR;
E1 and/or E2 binding modules are absent or are each independently selected from a heavy chain variable region (VH), a light chain variable region (VL), a single domain antibody, a VHH domain, a receptor ligand-binding domain, a ligand receptor-binding domain, a non-immunoglobulin antigen-binding scaffold, or a single variable domain of a TCR;
the C1 binding module and the D1 binding module are connected via a first linker peptide, and the C2 binding module and the D2 binding module are connected via a second linker peptide;
the first linker peptide and the second linker peptide comprise one or more cysteine residues (Cys), and the heterologous moiety is conjugated to the Cys residues of the first linker peptide and/or the second linker peptide;
the last cysteine residue (Cys) of the light chain constant region CL (position 214, EU numbering; all EU numbering hereinafter) is deleted (C214del).

2. The conjugate compound of claim 1, wherein the first linker peptide and the second linker peptide are polypeptides having a length of 5 to 20 amino acids.

3. The conjugate compound of claim 2, wherein the first linker peptide and the second linker peptide are flexible peptides or rigid peptides.

4. The conjugate compound of claim 3, wherein the flexible peptide consists of Cys and glycine (Gly), consists of Cys and serine (Ser), or consists of Cys, Ser, and Gly.

5. The conjugate compound of claim 3, wherein the rigid peptide consists of an α-helix; preferably, the rigid peptide consists of Cys, glutamic acid (Glu), alanine (Ala), and/or lysine (Lys).

6. The conjugate compound of claim 2, wherein the first linker peptide and the second linker peptide comprise the amino acid sequence as set forth in SEQ ID NO: 137: EPKSCDKTHTCPPCP (E216-P230).

7. The conjugate compound of claim 2, wherein one or two Cys residues at positions 220, 226, and/or 229 in SEQ ID NO: 137 of the first linker peptide and the second linker peptide are deleted and/or mutated; preferably, the Cys is mutated to Ser, Gly, or Thr.

8. The conjugate compound of claim 7, wherein the first linker peptide and the second linker peptide comprise the amino acid sequence as set forth in SEQ ID NO: 138, 139, or 140.

9. The conjugate compound of claim 2, wherein the first linker peptide and the second linker peptide comprise the amino acid sequence as set forth in SEQ ID NO: 156: ERKCCVECPPCP (E216-P230) (hinge20 IgG2 WT).

10. The conjugate compound of claim 2, wherein one or two Cys residues at positions 219, 220, 226, and/or 229 in SEQ ID NO: 156 of the first linker peptide and the second linker peptide are deleted and/or mutated; preferably, the Cys is mutated to Ser, Gly, or Thr.

11. The conjugate compound of claim 10, wherein the first linker peptide and the second linker peptide comprise the amino acid sequence as set forth in SEQ ID NO: 157, 158, 159, 160, 161, 162, 163, or 164.

12. The conjugate compound of claim 2, wherein the first linker peptide and the second linker peptide comprise the amino acid sequence as set forth in SEQ ID NO: 150: ESKYGPPCPPCP (E216-P230).

13. The conjugate compound of claim 2, wherein one of the Cys residues at positions 226 and 229 in SEQ ID NO: 150 of the first linker peptide and the second linker peptide is deleted or mutated; preferably, the Cys is mutated to Ser, Gly, or Thr.

14. The conjugate compound of claim 13, wherein the first linker peptide and the second linker peptide comprise the amino acid sequence as set forth in SEQ ID NO: 151, 152, 154, or 155.

15. The conjugate compound of any one of claims 1-14, wherein the first Fc and the second Fc comprise modified CH3 domains, and the modified CH3 domains comprise amino acid substitutions that promote heterodimerization of the first Fc and the second Fc.

16. The conjugate compound of claim 15, wherein one of the first Fc and the second Fc comprises the amino acid substitution T366W, and the other of the first Fc and the second Fc comprises the amino acid substitutions T366S, L368A, and Y407V.

17. The conjugate compound of claim 16, wherein the one of the first Fc and the second Fc comprising the amino acid substitution T366W further comprises the amino acid substitution S354C, and the other of the first Fc and the second Fc comprising the amino acid substitutions T366S, L368A, and Y407V further comprises the amino acid substitution Y349C.

18. The conjugate compound of claim 15, wherein one of the first Fc and the second Fc comprises the amino acid substitutions E356K and R409K, and the other of the first Fc and the second Fc comprises the amino acid substitutions R409K and K439E.

19. The conjugate compound of any one of claims 1-18, wherein the heterologous moiety is a substance suitable for disease diagnosis, cure, alleviation, treatment, or prevention, or a substance suitable for tumor targeting; preferably, the heterologous moiety is a drug, toxin, CDK inhibitor, HDAC inhibitor, TLR agonist, PROTAC protein degrader, radionuclide, immunomodulator, glutamate urea (DUPA) or a analog thereof, cytokine, lymphokine, chemokine, growth factor, tumor necrosis factor, hormone, hormone antagonist, enzyme, oligonucleotide, DNA, RNA, siRNA, RNAi, microRNA, peptide nucleic acid, phototherapeutic agent, anti-angiogenic agent, pro-apoptotic agent, non-natural amino acid, peptide, lipid, carbohydrate, scaffold molecule, fluorescent label, visualization peptide, biotin, serum half-life modifier, capture tag, chelator, or a combination thereof.

20. The conjugate compound of claim 19, wherein the heterologous moiety is an ethyleneimine derivative, triazene, folic acid analog, anthracycline, taxane, COX-2 inhibitor, pyrimidine analog, purine analog, antibiotic, enzyme inhibitor, epipodophyllotoxin, platinum coordination compound, vinca alkaloid, substituted urea, adrenocortical suppressant, hormone antagonist, endostatin, camptothecin, camptothecin derivative, SN-38, doxorubicin, doxorubicin analog, antimetabolite, alkylating agent, antimitotic agent, anti-angiogenic agent, mTOR inhibitor, heat shock protein inhibitor, proteasome inhibitor, HDAC inhibitor, pro-apoptotic agent, methotrexate, CPT-11, or a combination thereof.

21. The conjugate compound of claim 19, wherein the heterologous moiety is a nitrogen mustard, alkyl sulfonate, nitrosourea, gemcitabine, methylhydrazine derivative, paclitaxel, tyrosine kinase inhibitor, or a combination thereof.

22. The conjugate compound of claim 19, wherein the drug is auristatin, monomethyl auristatin E (MMAE), monomethyl auristatin F (MMAF), Dinaciclib, Mocetinostat, vorinostat, olaparib, Ceralasertib, M3541, CC-885, Dx-8951, tubulysin, exatecan derivative (Dxd), pyrrolobenzodiazepine (PBD), maytansinoid, DM1, DM4, calicheamicin, duocarmycin (CAS NO. 130288), duostatin, duostatin-3, duostatin-5, rachelmycin (CC-1065), SN-38, SG3199, or doxorubicin.

23. The conjugate compound of claim 19, wherein the heterologous moiety is glutamate urea (DUPA).

24. The conjugate compound of any one of claims 1-23, wherein the heterologous moiety is conjugated to the Cys residues of the first linker peptide and/or the second linker peptide via a linker.

25. The conjugate compound of claim 24, wherein the linker is a peptide linker, a non-peptide linker, a cleavable linker, or a non-cleavable linker.

26. The conjugate compound of claim 25, wherein the linker is selected from mc (maleimidocaproyl), val-cit (valine-citrulline), mc-val-cit (maleimidocaproyl-valine-citrulline), mc-val-cit-PABC (maleimidocaproyl-valine-citrulline-p-aminobenzyl carbamate), mcGGFG, mc-(PEG)₈-VA-PABC, MCC (N-maleimidomethyl cyclohexane-1-carboxylate), Mal-PEG2C2 (maleimido-[CH₂CH₂O]₂CH₂CH₂C(=O)), Mal-PEG3C2 (maleimido-[CH₂CH₂O]₃CH₂CH₂C(=O)), and Mal-PEG6C2 (maleimido-[CH₂CH₂O]₆CH₂CH₂C(=O)).

27. A method for preparing the conjugate compound of any one of claims 1-26, comprising placing the antibody moiety of any one of claims 1-26 under reducing conditions such that thiol groups of one or more Cys residues in the first linker peptide and the second linker peptide are reduced, and then reacting the reduced thiol groups with the heterologous moiety.

28. The method of claim 27, wherein the thiol groups are reacted with the heterologous moiety via a Michael reaction using a linker comprising a maleimide group.

29. A composition comprising the conjugate compound of any one of claims 1-26, and a pharmaceutically acceptable carrier, diluent, or excipient.

30. Use of the composition of claim 29 in the manufacture of a medicament for the prevention or treatment of a disease.

31. The use of claim 30, wherein the disease to be prevented or treated is selected from: cancer (e.g., breast cancer, lung cancer, prostate cancer, colon cancer, rectal cancer, bladder cancer, kidney cancer, liver cancer, thyroid cancer, endometrial cancer, muscle cancer, bone cancer, mesothelioma, vascular cancer, fibrotic cancer, leukemia, or lymphoma), autoimmune disease, inflammatory disease, or infectious disease.
